(19) Europäisches Patentamt / European Patent Office / Office européen des brevets

(11) EP 1 725 567 B1

(12) # EUROPÄISCHE PATENTSCHRIFT

(45) Veröffentlichungstag und Bekanntmachung des Hinweises auf die Patenterteilung:
**26.03.2008 Patentblatt 2008/13**

(21) Anmeldenummer: **04804136.2**

(22) Anmeldetag: **21.12.2004**

(51) Int Cl.:
***C07D 495/10*** (2006.01) ***C07D 491/04*** (2006.01)
***A61K 31/407*** (2006.01) ***A61P 29/00*** (2006.01)
***C07D 471/10*** (2006.01)

(86) Internationale Anmeldenummer:
**PCT/EP2004/014539**

(87) Internationale Veröffentlichungsnummer:
**WO 2005/066183 (21.07.2005 Gazette 2005/29)**

(54) **SPIROCYCLISCHE CYCLOHEXAN-DERIVATE MIT AFFINITÄT ZUM ORL1-REZEPTOR**
SPIROCYCLIC CYCLOHEXANE DERIVATIVES WITH AFFINITY TO THE ORL-1 RECEPTOR
DERIVES DE CYCLOHEXANE SPIROCYCLIQUES AVEC AFFINITE POUR LE RECEPTEUR ORL-1

(84) Benannte Vertragsstaaten:
**AT BE BG CH CY CZ DE DK EE ES FI FR GB GR HU IE IS IT LI LT LU MC NL PL PT RO SE SI SK TR**
Benannte Erstreckungsstaaten:
**HR LV**

(30) Priorität: **23.12.2003 DE 10360792**

(43) Veröffentlichungstag der Anmeldung:
**29.11.2006 Patentblatt 2006/48**

(73) Patentinhaber: **Grünenthal GmbH**
**52078 Aachen (DE)**

(72) Erfinder:
- **HINZE, Claudia**
**52066 Aachen (DE)**
- **SUNDERMANN, Bernd**
**52074 Aachen (DE)**
- **SCHICK, Hans**
**13086 Berlin-Weißensee (DE)**
- **HENKEL, Birgitta**
**12555 Berlin (DE)**
- **ENGLBERGER, Werner**
**52222 Stolberg (DE)**
- **OBERBÖRSCH, Stefan**
**52074 Aachen (DE)**
- **FRIDERICHS, Elmar**
**52223 Stolberg (DE)**
- **FRORMANN, Sven**
**52066 Aachen (DE)**
- **KÖGEL, Babette-Yvonne**
**52379 Langerwehe (DE)**
- **LINZ, Klaus**
**53343 Wachtberg (DE)**
- **MERLA, Beatrix**
**52078 Aachen (DE)**
- **SAUNDERS, Derek**
**52072 Aachen (DE)**
- **SCHRÖDER, Wolfgang**
**52074 Aachen (DE)**

(56) Entgegenhaltungen:
**EP-A- 0 466 548** **EP-A- 1 142 587**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft spirocyclische Cyclohexan-Derivate, Verfahren zu deren Herstellung, Arzneimittel enthaltend diese Verbindungen und die Verwendung von spirocyclischen Cyclohexan-Derivaten zur Herstellung von Arzneimitteln.

Das Heptadekapeptid Nociceptin ist ein endogener Ligand des ORL1 (Opioid-Receptor-Like)-Rezeptors (Meunier et al., Nature 377, 1995, S. 532-535), der zu der Familie der Opioid Rezeptoren gehört und in vielen Regionen des Gehirns und des Rückenmarks zu finden ist und eine hohe Affinität für den ORL1-Rezeptor aufweist. Der ORL1-Rezeptor ist homolog zu den μ, κ und δ Opioid-Rezeptoren und die Aminosäuresequenz des Nociceptin-Peptids weist eine starke Ähnlichkeit mit denen der bekannten Opioidpeptide auf. Die durch das Nociceptin induzierte Aktivierung des Rezeptors führt über die Kopplung mit $G_{i/o}$-Proteinen zu einer Inhibierung der Adenylatcyclase (Meunier et al., Nature 377, 1995, S. 532-535).

Das Nociceptin-Peptid zeigt nach intercerebroventricularer Applikation eine pronociceptive und hyperalgetische Aktivität in verschiedenen Tiermodellen (Reinscheid et al., Science 270, 1995, S. 792-794). Diese Befunde können als Hemmung der stressinduzierten Analgesie erklärt werden (Mogil et al., Neuroscience 75, 1996, S. 333-337). In diesem Zusammenhang konnte auch eine anxiolytische Aktivität des Nociceptin nachgewiesen werden (Jenck et al., Proc. Natl. Acad. Sci. USA 94, 1997, 14854-14858).

Auf der anderen Seite konnte in verschiedenen Tiermodellen, insbesondere nach intrathekaler Applikation, auch ein antinociceptiver Effekt von Nociceptin gezeigt werden. Nociceptin wirkt antinociceptiv in verschiedenen Schmerzmodellen, beispielsweise im Tail Flick-Test in der Maus (King et al., Neurosci. Lett., 223, 1997, 113-116. In Modellen für neuropathische Schmerzen konnte ebenfalls eine antinociceptive Wirkung von Nociceptin nachgewiesen, die insofern besonders interessant ist, als dass die Wirksamkeit von Nociceptin nach Axotomie von Spinalnerven zunimmt. Dies steht im Gegensatz zu den klassischen Opioiden, deren Wirksamkeit unter diesen Bedingungen abnimmt (Abdulla und Smith, J. Neurosci., 18, 1998, S. 9685-9694).

Der ORL1-Rezeptor ist außerdem noch an der Regulation weiterer physiologischer und pathophysiologischer Prozesse beteiligt. Hierzu gehören unter anderem Lernen und Gedächtnisbildung (Manabe et al., Nature, 394, 1997, S. 577-581), Hörvermögen (Nishi et al., EMBO J., 16, 1997, S. 1858-1864) sowie zahlreiche weitere Prozesse. In einem Übersichtsartikel von Calo et al. (Br.J. Pharmacol., 129, 2000, 1261 - 1283) wird ein Überblick über die Indikationen oder biologischen Vorgänge gegeben, in denen der ORL1-Rezeptor eine Rolle spielt oder mit hoher Wahrscheinlichkeit spielen könnte. Genannt werden u.a.: Analgesie, Stimulation und Regulation der Nahrungsaufnahme, Einfluß auf μ-Agonisten wie Morphin, Behandlung von Entzugserscheinungen, Reduzierung des Suchtpotentials von Opioiden, Anxiolyse, Modulation der Bewegungsaktivität, Gedächtnis-Störungen, Epilepsie; Modulation der Neurotransmitter-Ausschüttung, insbesondere von Glutamat, Serotonin und Dopamin, und damit neurodegenerative Erkrankungen; Beeinflußung des cardiovaskulären Systems, Auslösung einer Erektion, Diurese, Antinatriurese, Elektrolyt-Haushalt, arterieller Blutdruck, Wasserspeicher-Krankheiten, intestinale Motilität (Diarrhoe), relaxierende Effekte auf die Atemwege, Mikturations Reflex (Harninkontinenz). Weiter wird die Verwendung von Agonisten und Antagonisten als Anoretika, Analgetika (auch in Coadministration mit Opioiden) oder Nootropika diskutiert.

Entsprechend vielfältig sind die Anwendungsmöglichkeiten von Verbindungen, die an den ORL1-Rezeptor binden und diesen aktivieren oder inhibieren. Neben diesem spielen aber gerade im Bereich der Schmerztherapie aber auch anderen der genannten Indikationen Opioidrezeptoren wie der μ-Rezeptor, aber auch die anderen Subtypen dieser Opioidrezeptoren, nämlich δ und κ eine große Rolle. Entsprechend ist es günstig, wenn die Verbindung auch Wirkung an diesen Opioidrezeptoren zeigen.

EP 466 548 offenbart spirocyclische cyclohexan-Derivate für die Behandlung von ZNS-Erkrankungen.

Aufgabe der vorliegenden Erfindung war es, Arzneimittel zur Verfügung zu stellen, die auf das Nociceptin/ORL1-Rezeptor-System wirken und damit für Arzneimittel insbesondere zur Behandlung der verschiedenen mit diesem System nach dem Stand der Technik in Verbindung stehenden Krankeiten bzw. zum Einsatz in den dort genannten Indikationen geeignet sind.

Gegenstand der Erfindung sind daher spirocyclische Cyclohexan-Derivate der allgemeinen Formel I,

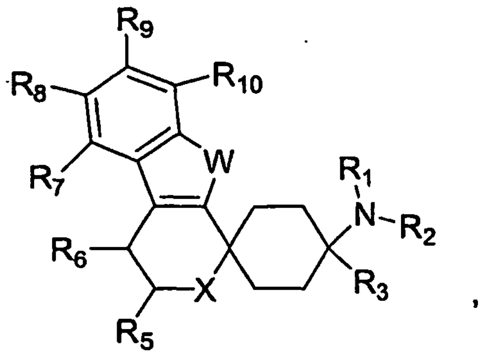

I

worin

$R^1$ und $R^2$, unabhängig voneinander für H; CHO; $C_{1-5}$-Alkyl jeweils gesättigt oder ungesättigt, verzweigt oder unverzweigt, einfach oder mehrfach substituiert oder unsubstituiert; $C_{3-8}$-Cycloalkyl, jeweils gesättigt oder ungesättigt, einfach oder mehrfach substituiert oder unsubstituiert; oder über $C_{1-3}$-Alkyl gebundenes Aryl, $C_{3-8}$-Cycloalkyl oder Heteroaryl, jeweils einfach oder mehrfach substituiert oder unsubstituiert, stehen;

oder die Reste $R^1$ und $R^2$ zusammen für $CH_2CH_2OCH_2CH_2$, $CH_2CH_2NR^{11}CH_2CH_2$ oder $(CH_2)_{3-6}$ stehen,

wobei $R^{11}$ H; $C_{1-5}$-Alkyl, jeweils gesättigt oder ungesättigt, verzweigt oder unverzweigt, einfach oder mehrfach substituiert oder unsubstituiert; $C_{3-8}$-Cycloalkyl, jeweils gesättigt oder ungesättigt, einfach oder mehrfach substituiert oder unsubstituiert; Aryl-, oder Heteroaryl, jeweils einfach oder mehrfach substituiert oder unsubstituiert; oder über $C_{1-3}$-Alkyl gebundenes Aryl, $C_{3-8}$-Cycloalkyl oder Heteroaryl, jeweils einfach oder mehrfach substituiert oder unsubstituiert, bedeutet;

$R^3$ für Heteroaryl, jeweils unsubstituiert oder einfach oder mehrfach substituiert, steht;

W für $NR^4$, O oder S steht

und

$R^4$ für H; $C_{1-5}$-Alkyl, gesättigt oder ungesättigt, verzweigt oder unverzweigt, unsubstituiert oder einfach oder mehrfach substituiert; Aryl, oder Heteroaryl, jeweils substituiert oder unsubstituiert; über eine $C_{1-3}$-Alkyl-Gruppe gebundenes Aryl, Heteroaryl oder Cycloalkyl, jeweils einfach oder mehrfach substituiert oder unsubstituiert; $COR^{12}$; $SO_2R^{12}$ steht,

wobei $R^{12}$ H; $C_{1-5}$-Alkyl, jeweils gesättigt oder ungesättigt, verzweigt oder unverzweigt, einfach oder mehrfach substituiert oder unsubstituiert; $C_{3-8}$-Cycloalkyl, jeweils gesättigt oder ungesättigt, einfach oder mehrfach substituiert oder unsubstituiert; Aryl-, oder Heteroaryl, jeweils einfach oder mehrfach substituiert oder unsubstituiert; oder über $C_{1-3}$-Alkyl gebundenes Aryl, $C_{3-8}$-Cycloalkyl oder Heteroaryl, jeweils einfach oder mehrfach substituiert oder unsubstituiert; $OR^{13}$; $NR^{14}R^{15}$ bedeutet;

$R^5$ für =O; H; $C_{1-5}$-Alkyl, gesättigt oder ungesättigt, verzweigt oder unverzweigt, unsubstituiert oder einfach oder mehrfach substituiert; $COOR^{13}$, $CONR^{13}$, $OR^{13}$; $C_{3-8}$-Cycloalkyl, gesättigt oder ungesättigt, unsubstituiert oder einfach oder mehrfach substituiert; Aryl-, oder Heteroaryl, unsubstituiert oder einfach oder mehrfach substituiert; oder über $C_{1-3}$-Alkyl gebundenes Aryl, $C_{3-8}$-Cycloalkyl oder Heteroaryl, unsubstituiert oder einfach oder mehrfach substituiert, steht;

$R^6$ für H; F, Cl, $NO_2$, $CF_3$, $OR^{13}$, $SR^{13}$, $SO_2R^{13}$, $SO_2OR^{13}$, CN, $COOR^{13}$, $NR^{14}R^{15}$; $C_{1-5}$-Alkyl, gesättigt oder ungesättigt, verzweigt oder unverzweigt, unsubstituiert oder einfach oder mehrfach substituiert; $C_{3-8}$-Cycloalkyl, gesättigt oder ungesättigt, unsubstituiert oder einfach oder mehrfach substituiert; Aryl-, oder Heteroaryl, unsubstituiert oder einfach oder mehrfach substituiert; oder über $C_{1-3}$-Alkyl gebundenes Aryl, $C_{3-8}$-Cycloalkyl oder Heteroaryl, unsubstituiert oder einfach oder mehrfach substituiert, steht;

oder $R^5$ und $R^6$ gemeinsam $(CH_2)_n$ mit n = 2, 3, 4, 5 oder 6 bedeuten, wobei einzelne Wasserstoffatome auch durch F, Cl, Br, I, $NO_2$, $CF_3$, $OR^{13}$, CN oder $C_{1-5}$-Alkyl ersetzt sein können;

$R^7$, $R^8$, $R^9$ und $R^{10}$ unabhängig voneinander für

H, F, Cl, Br, I, $NO_2$, $CF_3$, $OR^{13}$, $SR^{13}$, $SO_2R^{13}$, $SO_2OR^{13}$, CN, $COOR^{13}$, $NR^{14}R^{15}$; $C_{1-5}$-Alkyl, $C_{3-8}$-Cycloalkyl, unsubstituiert oder einfach oder mehrfach substituiert; Aryl-, oder Heteroaryl, unsubstituiert oder einfach oder mehrfach substituiert; oder über $C_{1-3}$-Alkyl gebundenes Aryl, $C_{3-8}$-Cycloalkyl oder Heteroaryl, unsubstituiert oder einfach oder mehrfach substituiert, stehen;

wobei $R^{13}$ H; $C_{1-5}$-Alkyl jeweils gesättigt oder ungesättigt, verzweigt oder unverzweigt, unsubstituiert oder einfach oder mehrfach substituiert; $C_{3-8}$-Cycloalkyl, jeweils gesättigt oder ungesättigt, unsubstituiert oder einfach oder mehrfach substituiert; Aryl-, oder Heteroaryl, unsubstituiert oder einfach oder mehrfach substituiert; oder über $C_{1-3}$-Alkyl gebundenes Aryl, $C_{3-8}$-Cycloalkyl oder Heteroaryl, unsubstituiert oder einfach oder mehrfach substituiert, bedeutet;

$R^{14}$ und $R^{15}$ unabhängig voneinander H; $C_{1-5}$-Alkyl , jeweils gesättigt oder ungesättigt, verzweigt oder unverzweigt,

unsubstituiert oder einfach oder mehrfach substituiert; oder $C_{3-8}$-Cycloalkyl, jeweils gesättigt oder ungesättigt, unsubstituiert oder einfach oder mehrfach substituiert; Aryl-, oder Heteroaryl, unsubstituiert oder einfach oder mehrfach substituiert; oder über $C_{1-3}$-Alkyl gebundenes Aryl, $C_{3-8}$-Cycloalkyl oder Heteroaryl, unsubstituiert oder einfach oder mehrfach substituiert, bedeuten;

oder $R^{14}$ und $R^{15}$ zusammen $CH_2CH_2OCH_2CH_2$, $CH_2CH_2NR^{16}CH_2CH_2$ oder $(CH_2)_{3-6}$ bilden,

wobei $R^{16}$ H; $C_{1-5}$-Alkyl gesättigt oder ungesättigt, verzweigt oder unverzweigt, unsubstituiert oder einfach oder mehrfach substituiert, bedeutet;

X für O, S, SO, $SO_2$ oder $NR^{17}$ steht;

$R^{17}$ für H; $C_{1-5}$-Alkyl, gesättigt oder ungesättigt, verzweigt oder unverzweigt; $COR^{12}$ oder $SO_2R^{12}$,

in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren oder Kationen.

Bei der Zusammenfassung verschiedener Reste, beispielsweise $R^7$, $R^8$, $R^9$ und $R^{10}$ sowie der Zusammenfassung von Resten an deren Substituenten, wie z. B. $OR^{13}$, $SR^{13}$, $SO_2R^{13}$ oder $COOR^{13}$, kann ein Substituent, z.B. $R^{13}$, für zwei oder mehrere Reste, beispielsweise $R^7$, $R^8$, $R^9$ und $R^{10}$, innerhalb einer Substanz unterschiedliche Bedeutungen annehmen.

Die erfindungsgemäßen Verbindungen zeigen gute Bindung an den ORL1-Rezeptor, aber auch an andere Opioidrezeptoren.

Die Ausdrücke "$C_{1-5}$-Alkyl" und "$C_{1-3}$-Alkyl" umfassen im Sinne dieser Erfindung acyclische gesättigte oder ungesättigte Kohlenwasserstoffreste, die verzweigt- oder geradkettig sowie unsubstituiert oder ein- oder mehrfach substituiert sein können, mit 1, 2, 3, 4 oder 5 C-Atomen bzw. 1, 2 oder 3 C-Atomen, d.h. $C_{1-5}$-Alkanyle, $C_{2-5}$-Alkenyle und $C_{2-5}$-Alkinyle bzw. $C_{1-3}$-Alkanyle, $C_{2-3}$-Alkenyle und $C_{2-3}$-Alkinyle. Dabei weisen Alkenyle mindestens eine C-C-Doppelbindung und Alkinyle mindestens eine C-C-Dreifachbindung auf. Vorteilhaft ist Alkyl aus der Gruppe ausgewählt, die Methyl, Ethyl, n-Propyl, 2-Propyl, n-Butyl, iso-Butyl, sec.-Butyl, tert.-Butyl, n-Pentyl, iso-Pentyl, neo-Pentyl, n-Hexyl, 2-Hexyl; Ethylenyl (Vinyl), Ethinyl, Propenyl ($-CH_2CH=CH_2$, $-CH=CH-CH_3$, $-C(=CH_2)-C_{H3}$), Propinyl ($-CH-C\equiv CH$,$-C\equiv C-CH_3$), 1,1-Dimethylethyl, 1,1-Dimethylpropyl, Butenyl, Butinyl, Pentenyl und Pentinyl, umfaßt.

Der Ausdruck "Cycloalkyl" oder "$C_{3-8}$-Cycloalkyl" bedeutet für die Zwecke dieser Erfindung cyclische Kohlenwasserstoffe mit 3, 4, 5, 6, 7 oder 8 Kohlenstoffatomen, wobei die Kohlenwasserstoffe gesättigt oder ungesättigt (aber nicht aromatisch), unsubstituiert oder ein- oder mehrfach substituiert sein können. In Bezug auf Cycloalkyl umfasst der Begriff auch gesättigte oder ungesättigte (aber nicht aromatische) Cycloalkyle, in denen ein oder zwei Kohlenstoffatome durch ein Heteroatom S, N oder O ersetzt sind. Vorteilhaft ist $C_{3-8}$-Cycloalkyl aus der Gruppe ausgewählt, die Cyclopropyl, Cyclobutyl, Cyclopentyl, Cyclohexyl, Cycloheptyl, Cyclooctyl, Cyclopentenyl, Cyclohexenyl, Cycloheptenyl und Cyclooctenyl, aber auch Tetrahydropyranyl, Dioxanyl, Dioxolanyl, Morpholinyl, Piperidinyl, Piperazinyl, Pyrazolinonyl und Pyrrolidinyl enthält.

Unter dem Begriff $(CH_2)_{3-6}$ ist $-CH_2-CH_2-CH_2-$, $-CH_2-CH_2-CH_2-CH_2-$, $-CH_2-CH_2-CH_2-CH_2-CH_2-$ und $CH_2-CH_2-CH_2-CH_2-CH_2-CH_2-$ zu verstehen.

Der Ausdruck "Aryl" bedeutet im Sinne dieser Erfindung carbocyclische Ringsysteme mit mindestens einem aromatischen Ring, aber ohne Heteroatome in nur einem der Ringe, u.a. Phenyle, Naphthyle und Phenanthrenyle, Fluoranthenyle, Fluorenyle, Indanyle und Tetralinyle. Die Aryl-Reste können auch mit weiteren gesättigten, (partiell) ungesättigten oder aromatischen Ringsystemen kondensiert sein. Jeder Aryl-Rest kann unsubstituiert oder einfach oder mehrfach substituiert vorliegen, wobei die Aryl-Substituenten gleich oder verschieden und in jeder beliebigen und möglichen Position des Aryls sein können. Besonders vorteilhaft sind Phenyl- oder Naphthyl-Reste.

Der Ausdruck "Heteroaryl" steht für einen 5-, 6- oder 7-gliedrigen cyclischen aromatischen Rest, der mindestens 1, ggf. auch 2, 3, 4 oder 5 Heteroatome, enthält, wobei die Heteroatome gleich oder verschieden sind und der Heterocyclus unsubstituiert oder ein- oder mehrfach substituiert sein kann; im Falle der Substitution am Heterocyclus können die Substituenten gleich oder verschieden sein und in jeder beliebigen und möglichen Position des Heteroaryls sein. Der Heterocyclus kann auch Teil eines bi- oder polycyclischen Systems sein. Bevorzugte Heteroatome sind Stickstoff, Sauerstoff und Schwefel. Es ist bevorzugt, daß der Heteroaryl-Rest ausgewählt ist aus der Gruppe, die Pyrrolyl, Indolyl, Furyl (Furanyl), Benzofuranyl, Thienyl (Thiophenyl), Benzothienyl, Benzothiadiazolyl, Benzothiazolyl, Benzotriazolyl, Benzodioxolanyl, Benzodioxanyl, Phtalazinyl, Pyrazolyl, Imidazolyl, Thiazolyl, Oxazolyl, Isoxazolyl, Pyridinyl, Pyridazinyl, Pyrimidinyl, Pyrazinyl, Pyranyl, Indazolyl, Purinyl, Indolizinyl, Chinolinyl, Isochinolinyl, Chinazolinyl, Carbazolyl, Phenazinyl, Phenothiazinyl oder Oxadiazolyl enthält, wobei die Bindung an die Verbindungen der allgemeinen Struktur I über jedes beliebige und mögliche Ringglied des Heteröaryl-Restes erfolgen kann.

Im Zusammenhang mit "Alkyl" versteht man unter dem Begriff "substituiert" im Sinne dieser Erfindung die Substitution eines oder mehrerer Wasserstoffreste durch F, Cl, Br, I, -CN, $NH_2$, NH-Alkyl, NH-Aryl, NH-Heteroaryl, NH-Cycloalkyl, NH-Alkyl-Aryl, NH-Alkyl-Heteroaryl, NH-Alkyl-OH, $N(Alkyl)_2$, $N(Alkyl-Aryl)_2$, $N(Alkyl-Heteroaryl)_2$, $N(Cycloalkyl)_2$, $N(Alkyl-OH)_2$, $NO_2$, SH, S-Alkyl, S-Aryl, S-Heteroaryl, S-Alkyl-Aryl, S-Alkyl-Heteroaryl, S-Cycloalkyl, S-Alkyl-OH, S-Alkyl-SH, OH, O-Alkyl, O-Aryl, 0-Heteroaryl, O-Alkyl-Aryl, O-Alkyl-Heteroaryl, O-Cycloalkyl, O-Alkyl-OH, CHO, C(=O)

$C_{1-6}$-Alkyl, C(=S)$C_{1-6}$-Alkyl, C(=O)Aryl, C(=S)Aryl, C(=O)$C_{1-6}$-Alkyl-Aryl, C(=S)$C_{1-6}$-Alkyl-Aryl, C(=O)-Heteroaryl, C (=S)-Heteroaryl, C(=O)-Cycloalkyl, C(=S)-Cycloalkyl, $CO_2H$, $CO_2$-Alkyl, $CO_2$-Alkyl-Aryl, C(=O)$NH_2$, C(=O)NH-Alkyl, C (=O)NHAryl, C(=O)NH-Cycloalkyl, C(=O)N(Alkyl)$_2$, C(=O)N(Alkyl-Aryl)$_2$, C(=O)N(Alkyl-Heteroaryl)$_2$, C(=O)N(Cycloalkyl)$_2$, SO-Alkyl, $SO_2$-Alkyl, $SO_2NH_2$, $SO_3H$, PO(O-$C_{1-6}$-Alkyl)$_2$, Si($C_{1-6}$-Alkyl)$_3$, Si($C_{3-8}$-Cycloalkyl)$_3$, Si($CH_2$-$C_{3-8}$-Cycloalkyl)$_3$, Si(Phenyl)$_3$, Cycloalkyl, Aryl oder Heteroaryl, wobei unter mehrfach substituierten Resten solche Reste zu verstehen sind, die entweder an verschiedenen oder an gleichen Atomen mehrfach, z. B. zwei- oder dreifach, substituiert sind, beispielsweise dreifach am gleichen C-Atom wie im Falle von $CF_3$ oder -$CH_2CF_3$ oder an verschiedenen Stellen wie im Falle von -CH(OH)-CH=CH-$CHCl_2$. Die Mehrfachsubstitution kann mit dem gleichen oder mit verschiedenen Substituenten erfolgen. Ggf. kann ein Substituent auch seinerseits substituiert sein; so umfaßt -OAlkyl u.a. auch -O-$CH_2$-$CH_2$-O-$CH_2$-$CH_2$-OH.

In Bezug auf "Aryl", "Heteroaryl" sowie "Cycloalkyl" versteht man im Sinne dieser Erfindung unter "ein- oder mehrfach substituiert" die ein- oder mehrfache, z.B. zwei-, drei- vier- oder fünffache, Substitution eines oder mehrerer Wasserstoffatome des Ringsystems durch F, Cl, Br, I, CN, $NH_2$, NH-Alkyl, NH-Aryl, NH-Heteroaryl, NH-Alkyl-Aryl, NH-Alkyl-Heteroaryl, NH-Cycloalkyl, NH-Alkyl-OH, N(Alkyl)$_2$, N(Alkyl-Aryl)$_2$, N(Alkyl-Heteroaryl)$_2$, N(Cycloalkyl)$_2$, N(Alkyl-OH)$_2$, $NO_2$, SH, S-Alkyl, S-Cycloalkyl, S-Aryl, S-Heteroaryl, S-Alkyl-Aryl, S-Alkyl-Heteroaryl, S-Alkyl-OH, S-Alkyl-SH, OH, O-Alkyl, O-Cycloalkyl, O-Aryl, O-Heteroaryl, O-Alkyl-Aryl, O-Alkyl-Heteroaryl, O-Alkyl-OH, CHO, C(=O)$C_{1-6}$-Alkyl, C(=S)$C_{1-6}$-Alkyl, C(=O)Aryl, C(=S)Aryl, C(=O)-$C_{1-6}$-Alkyl-Aryl, C(=S)$C_{1-6}$-Alkyl-Aryl, C(=O)-Heteroaryl, C(=S)-Heteroaryl, C(=O)-Cycloalkyl, C(=S)-Cycloalkyl, $CO_2H$, $CO_2$-Alkyl, $CO_2$-Alkyl-Aryl, C(=O)$NH_2$, C(=O)NH-Alkyl, C(=O)NHAryl, C(=O)NH-Cycloalkyl, C(=O)N(Alkyl)$_2$, C(=O)N(Alkyl-Aryl)$_2$, C(=O)N(Alkyl-Heteroaryl)$_2$, C(=O)N(Cycloalkyl)$_2$, S(O)-Alkyl, S(O)-Aryl, $SO_2$-Alkyl, $SO_2$-Aryl, $SO_2NH_2$, $SO_3H$, $CF_3$, =O, =S; Alkyl, Cycloalkyl, Aryl und/oder Heteroaryl; an einem oder ggf. verschiedenen Atomen (wobei ein Substituent ggf. seinerseits substituiert sein kann). Die Mehrfachsubstitution erfolgt dabei mit dem gleichen oder mit unterschiedlichen Substituenten.

Unter dem Begriff Salz ist jegliche Form des erfindungsgemäßen Wirkstoffes zu verstehen, in dem dieser eine ionische Form annimmt bzw. geladen ist und mit einem Gegenion (einem Kation oder Anion) gekoppelt ist bzw. sich in Lösung befindet. Darunter sind auch Komplexe des Wirkstoffes mit anderen Molekülen und Ionen zu verstehen, insbesondere Komplexe, die über ionische Wechselwirkungen komplexiert sind. Insbesondere versteht man darunter (und dies ist auch eine bevorzugte Ausführungsform dieser Erfindung) physiologisch verträgliche Salze, insbesondere physiologisch verträgliche Salze mit Kationen oder Basen und physiologisch verträgliche Salze mit Anionen oder Säuren oder auch ein mit einer physiologisch verträglichen Säure oder einem physiologisch verträglichen Kation gebildetes Salz.

Unter dem Begriff des physiologisch verträglichen Salzes mit Anionen oder Säuren versteht man im Sinne dieser Erfindung Salze mindestens einer der erfindungsgemäßen Verbindungen - meist, beispielsweise am Stickstoff, protoniert - als Kation mit mindestens einem Anion, die physiologisch - insbesondere bei Anwendung im Menschen und/oder Säugetier - verträglich sind. Insbesondere versteht man darunter im Sinne dieser Erfindung das mit einer physiologisch verträglichen Säure gebildete Salz, nämlich Salze des jeweiligen Wirkstoffes mit anorganischen bzw. organischen Säuren, die physiologisch - insbesondere bei Anwendung im Menschen und/oder Säugetier - verträglich sind. Beispiele für physiologisch verträgliche Salze bestimmter Säuren sind Salze der: Salzsäure, Bromwasserstoffsäure, Schwefelsäure, Methansulfonsäure, Ameisensäure, Essigsäure, Oxalsäure, Bernsteinsäure, Apfelsäure, Weinsäure, Mandelsäure, Fumarsäure, Milchsäure, Zitronensäure, Glutaminsäure, Saccharinsäure, Monomethylsebacinsäure, 5-Oxo-prolin, Hexan-1-sulfonsäure, Nicotinsäure, 2-, 3- oder 4-Aminobenzoesäure, 2,4,6-Trimethyl-benzoesäure, $\alpha$-Liponsäure, Acetylglycin, Hippursäure und/oder Asparaginsäure. Besonders bevorzugt ist das HydrochloridSalz, das Citrat und das Hemicitrat.

Unter dem Begriff des mit einer physiologisch verträglichen Säure gebildeten Salzes versteht man im Sinne dieser Erfindung Salze des jeweiligen Wirkstoffes mit anorganischen bzw. organischen Säuren, die physiologisch - insbesondere bei Anwendung im Menschen und/oder Säugetier - verträglich sind. Besonders bevorzugt ist das Hydrochlorid und das Citrat. Beispiele für physiologisch verträgliche Säuren sind: Salzsäure, Bromwasserstoffsäure, Schwefelsäure, Methansulfonsäure, Ameisensäure, Essigsäure, Oxalsäure, Bernsteinsäure, Weinsäure, Mandelsäure, . Fumarsäure, Milchsäure, Zitronensäure, Glutaminsäure, Saccharinsäure, Monomethylsebacinsäure, 5-Oxo-prolin, Hexan-1-sulfonsäure, Nicotinsäure, 2-, 3- oder 4-Aminobenzoesäure, 2,4,6-Trimethyl-benzoesäure, $\alpha$-Liponsäure, Acetylglycin, Acetylsalicylsäure, Hippursäure und/oder Asparaginsäure.

Unter dem Begriff des physiologisch verträglichen Salzes mit Kationen oder Basen versteht man im Sinne dieser Erfindung Salze mindestens einer der erfindungsgemäßen Verbindungen - meist einer (deprotonierten) Säure - als Anion mit mindestens einem, vorzugsweise anorganischen, Kation, die physiologisch - insbesondere bei Anwendung im Menschen und/oder Säugetier - verträglich sind. Besonders bevorzugt sind die Salze der Alkali- und Erdalkalimetalle aber auch Ammoniumsalze, insbesondere aber (Mono-) oder (Di-) Natrium-, (Mono-) oder (Di-) Kalium-, Magnesium- oder Calzium-Salze.

Unter dem Begriff des mit einem physiologisch verträglichen Kation gebildeten Salzes versteht man im Sinne dieser Erfindung Salze mindestens einer der jeweiligen Verbindungen als Anion mit mindestens einem anorganischen Kation, das physiologisch - insbesondere bei Anwendung im Menschen und/oder Säugetier - verträglich ist. Besonders

bevorzugt sind die Salze der Alkali- und Erdalkalimetalle aber auch Ammoniumsalze, insbesondere aber (Mono-) oder (Di-) Natrium-, (Mono-) oder (Di-) Kalium-, Magnesium- oder Calzium-Salze.

Für eine bevorzugte Ausführungsform der erfindungsgemäßen spirocyclischen Cyclohexan-Derivate gilt, dass $R^1$ und $R^2$, unabhängig voneinander für H; $C_{1-2}$-Alkyl jeweils gesättigt oder ungesättigt, verzweigt oder unverzweigt, jeweils einfach oder mehrfach substituiert oder unsubstituiert, stehen;
oder die Reste $R^1$ und $R^2$ zusammen für $CH_2CH_2OCH_2CH_2$, $CH_2CH_2NR^{11}$ $CH_2CH_2$ oder $(CH_2)_{3-6}$ stehen.

Für eine weitere bevorzugte Ausführungsform der erfindungsgemäßen spirocyclischen Cyclohexan-Derivate gilt, dass
$R^1$ und $R^2$ unabhängig voneinander für H, $C_{1-5}$-Alkyl, verzweigt oder unverzweigt, gesättigt oder ungesättigt, unsubstituiert oder einfach oder mehrfach substituiert, oder CHO stehen,
$R^3$ für Heteroaryl, unsubstituiert oder ein- oder mehrfach substituiert, steht
$R^5$ für H, $C_{1-5}$-Alkyl, verzweigt oder unverzweigt, unsubstituiert oder einfach oder mehrfach substituiert, $COOR^{13}$, steht
$R^6$ für H oder $C_{1-5}$-Alkyl steht,
$R^7$, $R^8$, $R^9$ und $R^{10}$ unabhängig voneinander für H; $C_{1-5}$-Alkyl, verzweigt oder unverzweigt, unsubstituiert oder ein- oder mehrfach substituiert; F, Cl, Br, I, OH, $OCH_3$, $NH_2$, COOH, $COOCH_3$, $NHCH_3$ oder $N(CH_3)_2$ oder $NO_2$ stehen.

Gemäß der Erfindung bevorzugt sind auch spirocyclische Cyclohexan-Derivate der allgemeinen Formel I, worin W für $NR^4$, O oder S steht, und X O, S, SO, $SO_2$ oder $NR^{17}$, vorzugsweise O oder N $R^{17}$, bedeutet,
$R^1$ und $R^2$ unabhängig voneinander für H; $C_{1-4}$-Alkyl, verzweigt oder unverzweigt, ein- oder mehrfach substituiert oder unsubstituiert; oder CHO stehen
$R^3$ für Heteroaryl, unsubstituiert oder einfach oder mehrfach substituiert, steht
$R^4$ für H; $C_{1-3}$-Alkyl, ein- oder mehrfach substituiert oder unsubstituiert; $CO(CH_2)_mH$ mit m = 0 bis 2, steht, und/oder
$R^5$ und $R^6$ jeweils für H stehen und/oder
$R^7$, $R^8$, $R^9$ und $R^{10}$ unabhängig voneinander für H; $C_{1-5}$-Alkyl, $OC_{1-3}$-Alkyl, jeweils verzweigt oder unverzweigt, gesättigt oder ungesättigt, unsubstituiert oder ein- oder mehrfach substituiert; F, Cl, Br, I, $CF_3$, OH, SH, $SCH_3$, $OCH_3$, $NH_2$, COOH, $COOCH_3$, $NHCH_3$ oder $N(CH_3)_2$ oder $NO_2$, stehen,
wobei Verbindungen, bei denen W für $NR^4$ und X für O steht besonders bevorzugt sind.

Für eine besonders bevorzugte Ausführungsform der erfindungsgemäßen spirocyclischen Cyclohexan-Derivate gilt, dass
$R^1$ und $R^2$ unabhängig voneinander H oder $CH_3$ bedeuten, wobei $R^1$ und $R^2$ nicht gleichzeitig H bedeuten.
Für eine besonders bevorzugte Ausführungsform der erfindungsgemäßen spirocyclischen Cyclohexan-Derivate gilt, dass
$R^3$ Thienyl oder Pyridyl bedeutet.

Für eine ganz besonders bevorzugte Ausführungsform der erfindungsgemäßen spirocyclischen Cyclohexan-Derivate gilt, dass
der Rest $R^5$ für H, $CH_3$, $COOCH_3$ oder $CH_2OH$ steht
der Rest $R^6$ für H steht
$R^7$ $R^8$, $R^9$ und $R^{10}$ unabhängig voneinander H; $C_{1-5}$-Alkyl, verzweigt oder unverzweigt, unsubstituiert oder ein- oder mehrfach substituiert; F, Cl, Br, I, $CF_3$, OH, $OCH_3$, $NH_2$, COOH, $COOCH_3$, $NHCH_3$ oder $N(CH_3)_2$ oder $NO_2$ bedeuten vorzugsweise
die Reste $R^6$, $R^7$, $R^8$, $R^9$ und $R^{10}$ für H stehen
oder
einer der Reste $R^6$, $R^7$ und $R^8$ für H; $C_{1-5}$-Alkyl, verzweigt oder unverzweigt, unsubstituiert oder ein- oder mehrfach substituiert; F, Cl, Br, I, OH, $OCH_3$, COOH, $COOCH_3$, $NH_2$, $NHCH_3$ oder $N(CH_3)_2$ oder $NO_2$ steht, während die übrigen Reste H sind,
oder
zwei der Reste $R^6$, $R^7$ $R^8$, $R^9$ und $R^{10}$ unabhängig voneinander für H; $C_{1-5}$-Alkyl, verzweigt oder unverzweigt, unsubstituiert oder ein- oder mehrfach substituiert; F, Cl, Br, I, OH, $OCH_3$, COOH, $COOCH_3$, $NH_2$, $NHCH_3$ oder $N(CH_3)_2$ oder $NO_2$ stehen, während die übrigen Reste H sind.

Weiterhin besonders bevorzugt sind Verbindungen, bei denen W $NR^4$, X O und $R^4$ H, $CH_3$, $C_2H_5$, Acetyl, Phenyl, Benzyl oder $COR^{12}$, insbesondere H bedeutet.

Für eine ganz besonders bevorzugte Ausführungsform der erfindungsgemäßen spirocyclischen Cyclohexan-Derivate gilt, dass
$R^1$ und $R^2$ unabhängig voneinander H oder $CH_3$, insbesondere $CH_3$ bedeuten, $R^3$ Pyridyl oder Thienyl bedeutet und/oder die Reste $R^5$, $R^6$, $R^7$, $R^9$ und $R^{10}$ H und der Rest $R^8$ H oder F bedeuten.

Ganz besonders bevorzugt sind spirocyclische Cyclohexan-Derivate aus der Gruppe

1,1-[3-Dimethylamino-3-(pyridin-2-yl)pentamethylen]-3,4-dihydro-1H-2,9-diazafluoren
2-Acetyl-1,1-[3-dimethylamino-3-(pyridin-2-yl)pentamethylen]-3,4-dihydro-1 H-2,9-diazafluoren

1,1-[3-Dimethylamino-3-(pyridin-2-yl)pentamethylen]-3,4-dihydro-1 H-2-oxa-9-thiafluoren

1,1-[3-Dimethylamino-3-(pyridin-2-yl)pentamethylen]-1,3,4,9-tetrahydropyrano-[3,4-b]indol Hemicitrat, unpolareres Diastereoisomer

1,1-[3-Dimethylamino-3-(pyridin-2-yl)pentamethylen]-1,3,4,9-tetrahydropyrano-[3,4-b]indol Citrat, polareres Diastereoisomer

1,1-[3-Dimethylamino-3-(2-thienyl)pentamethylen]-1,3,4,9-tetra-hydropyrano[3,4-b]indol Dimethansulfonat; polareres Diastereomer

1,1-[3-Dimethylamino-3-(2-thienyl)pentamethylen]-1,3,4,9-tetra-hydropyrano[3,4-b]indol Citrat; unpolareres Diastereomer

1,1-[3-Dimethylamino-3-(3-thienyl)pentamethylen]-1,3,4,9-tetra-hydropyrano[3,4-b]indol Hemicitrat; unpolareres Diastereomer

1,1-[3-Dimethylamino-3-(3-thienyl)pentamethylen]-1,3,4,9-tetra-hydropyrano[3,4-b]indol Citrat; polareres Diastereomer

1,1-[3-Dimethylamino-3-(2-thienyl)pentamethylen]-1,3,4,9-tetra-hydropyrano[3,4-b]-6-fluorindol Hemicitrat; unpolareres Diastereomer

1,1-[3-Dimethylamino-3-(2-thienyl)pentamethylen]-1,3,4,9-tetra-hydropyrano[3,4-b]-6-fluorindol Citrat; polareres Diastereomer

1,1-[3-Dimethylamino-3-(3-thienyl)pentamethylen]-1,3,4,9-tetra-hydropyrano[3,4-b]-6-fluorindol Dimethansulfonat; polareres Diastereomer

1,1-[3-Dimethylamino-3-(3-thienyl)pentamethylen]-1,3,4,9-tetra-hydropyrano[3,4-b]-6-fluorindol Hemicitrat; unpolareres Diastereomer

1,1-[3-Methylamino-3-(2-thienyl)pentamethylen]-1,3,4,9-tetrahydro-pyrano[3,4-b]indol Citrat

1,1-[3-Methylamino-3-(2-thienyl)pentamethylen]-1,3,4,9-tetrahydro-pyrano[3,4-b]-6-fluorindol Citrat

1,1-[3-Methylamino-3-(3-thienyl)pentamethylen]-1,3,4,9-tetrahydro-pyrano[3,4-b]indol Citrat

1,1-[3-Methylamino-3-(3-thienyl)pentamethylen]-1,3,4,9-tetrahydro-pyrano[3,4-b]-6-fluorindol Citrat

gegebenenfalls auch als Gemisch.

Die erfindungsgemäßen Substanzen wirken beispielsweise auf den im Zusammenhang mit verschiedenen Erkrankungen relevanten ORL1-Rezeptor, sodass sie sich als pharmazeutischer Wirkstoff in einem Arzneimittel eignen. Ein weiterer Gegenstand der Erfindung sind daher Arzneimittel enthaltend wenigstens ein erfindungsgemäßes spirocyclisches Cyclohexan-Derivat, sowie gegebenenfalls geeignete Zusatz- und/oder Hilfsstoffe und/oder gegebenenfalls weitere Wirkstoffe.

Die erfindungsgemäßen Arzneimittel enthalten neben mindestens einem erfindungsgemäßen spirocyclischen Cyclohexan-Derivat gegebenenfalls geeignete Zusatz- und/oder Hilfsstoffe, so auch Trägermaterialien, Füllstoffe, Lösungsmittel, Verdünnungsmittel, Farbstoffe und/oder Bindemittel und können als flüssige Arzneiformen in Form von Injektionslösungen, Tropfen oder Säfte, als halbfeste Arzneiformen in Form von Granulaten, Tabletten, Pellets, Patches, Kapseln, Pflaster/Sprühpflaster oder Aerosolen verabreicht werden. Die Auswahl der Hilfsstoffe etc. sowie die einzusetzenden Mengen derselben hängen davon ab, ob das Arzneimittel oral, peroral, parenteral, intravenös, intraperitoneal, intradermal, intramuskulär, intranasal, buccal, rektal oder örtlich, zum Beispiel auf die Haut, die Schleimhäute oder in die Augen, appliziert werden soll. Für die orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße spirocyclische Cyclohexan-Derivate in einem Depot, in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die erfindungsgemäßen spirocyclischen Cyclohexan-Derivate verzögert freisetzen. Die erfindungsgemäßen spirocyclischen Cyclohexan-Derivate können auch in parenteralen Langzeitdepotformen wie z. B. Implantaten oder implantierten Pumpen angewendet werden. Prinzipiell können den erfindungsgemäßen Arzneimitteln andere dem Fachmann bekannte weitere Wirkstoffe zugesetzt werden.

Die an den Patienten zu verabreichende Wirkstoffmenge variiert in Abhängigkeit vom Gewicht des Patienten, von der Applikationsart, der Indikation und dem Schweregrad der Erkrankung. Üblicherweise werden 0,00005 bis 50 mg/kg, bevorzugt 0,001 bis 0,5 mg/kg wenigstens eines erfindungsgemäßen spirocyclischen Cyclohexan-Derivats appliziert.

Für alle vorstehenden Formen der erfindungsgemäßen Arzneimittel ist es besonders bevorzugt, wenn das Arzneimittel neben wenigstens einem spirocyclischen Cyclohexan-Derivat noch einen weiteren Wirkstoff, insbesondere ein Opioid, vorzugsweise ein starkes Opioid, insbesondere Morphin, oder ein Anesthetikum, vorzugsweise Hexobarbital oder Halothan, enthält.

In einer bevorzugten Form des Arzneimittels liegt ein enthaltenes erfindungsgemäßes spirocyclisches Cyclohexan-Derivat als reines Diastereomer und/oder Enantiomer, als Razemat oder als nicht-äquimolare oder äquimolare

Mischung der Diastereomere und/oder Enantiomere vor.

Wie in der Einleitung am Stand der Technik abzulesen, wurde der ORL1-Rezeptor insbesondere im Schmerzgeschehen identifiziert. Entsprechend können erfindungsgemäße spirocyclische Cyclohexan-Derivate zur Herstellung eines Arzneimittels zur Behandlung von Schmerz, insbesondere von akutem, neuropathischem oder chronischem Schmerz, verwendet werden.

Ein weiterer Gegenstand der Erfindung ist daher die Verwendung eines erfindungsgemäßen spirocyclischen Cyclohexan-Derivats zur Herstellung eines Arzneimittels zur Behandlung von Schmerz, insbesondere von akutem, viszeralem, neuropathischem oder chronischem Schmerz.

In pharmakologischen Untersuchungen fiel auf, dass die erfindungsgemäßen Verbindungen besonders geeignet sind zur Behandlung von Opioid-Missbrauch, aber auch als Muskelrelaxans oder Anesthetikum einsetzbar sind. Ein weiterer Gegenstand der Erfindung ist daher die Verwendung eines erfindungsgemäßen spirocyclischen Cyclohexan-Derivats zur Herstellung eines Arzneimittels zu r Behandlung von Entzugserscheinungen, Alkohol- und/oder Drogen- und/oder Medikamentenmißbrauch und/oder -abhängigkeit, als Muskelrelaxanz oder Anesthetikum, bzw. zur Coadministration bei Behandlung mit einem opioiden Analgetikum oder mit einem Anesthetikum, zur Behandlung von Entzugserscheinungen und/oder zur Reduzierung des Suchtpotentials von Opioiden.

Ein weiterer Gegenstand der Erfindung ist die Verwendung eines erfindungsgemäßen spirocyclischen Cyclohexan-Derivats zur Herstellung eines Arzneimittels zur Behandlung von Angstzuständen, von Stress und mit Stress verbundenen Syndromen, Depressionen, Epilepsie, Alzheimer Erkrankung, seniler Demenz, allgemeinen kognitiven Dysfunktionen, Lern- und Gedächtnis-Störungen (als Nootropikum), sexuellen Dysfunktionen, cardiovaskulären Erkrankungen, Hypotension, Hypertension, Tinitus, Pruritus, Migräne, Schwerhörigkeit, mangelnder Darmmotilität, gestörter Nahrungsaufnahme, Anorexie, Fettsucht, lokomotorischen Störungen, Diarrhoe, Kachexie, Harninkontinenz bzw. als Antikonvulsivum, zur Diurese oder Antinatriurese, Anxiolyse, zur Modulation der Bewegungsaktivität und zur Modulation der Neurotransmitter-Ausschüttung und Behandlung damit verbundener neurodegenerativer Erkrankungen.

Dabei kann es in einer der vorstehenden Verwendungen bevorzugt sein, wenn ein verwendetes spirocyclisches Cyclohexan-Derivat als reines Diastereomer und/oder Enantiomer, als Razemat oder als nicht-äquimolare oder äquimolare Mischung der Diastereomere und/oder Enantiomere vorliegt.

Ein weiterer Gegenstand der Erfindung ist ein Verfahren zur Behandlung, insbesondere in einer der vorgenannten Indikationen, eines nichthumanen Säugetieres oder Menschen, das oder der eine Behandlung von Schmerzen, insbesondere chronischer Schmerzen, benötigt, durch Verabreichung einer therapeutisch wiksamen Dosis eines erfindungsgemäßen spirocyclischen Cyclohexan-Derivats, oder eines erfindungsgemäßen Arzneimittels.

Ein weiterer Gegenstand der Erfindung ist ein Verfahren zur Herstellung der erfindungsgemäßen spirocyclischen Cyclohexan-Derivate wie in der folgenden Beschreibung und Beispielen ausgeführt. Insbesondere geeignet ist dabei ein, im folgenden Hauptverfahren genanntes, Verfahren zur Herstellung eines erfindungsgemäßen spirocyclischen Cyclohexan-Derivats mit folgenden Schritten,

wobei X, W, $R^1$, $R^2$, $R^3$, $R^5$, $R^6$, $R^7$, $R^8$, $R^9$ und $R^{10}$ die für erfindungsgemäße Verbindungen gemäß Formel I angegebene Bedeutung haben,

und

$R^{01}$ und $R^{02}$ die für erfindungsgemäße Verbindungen gemäß Formel I für $R^1$ und $R^2$ angegebene Bedeutung haben und zusätzlich unabhängig voneinander für eine Schutzgruppe stehen können:

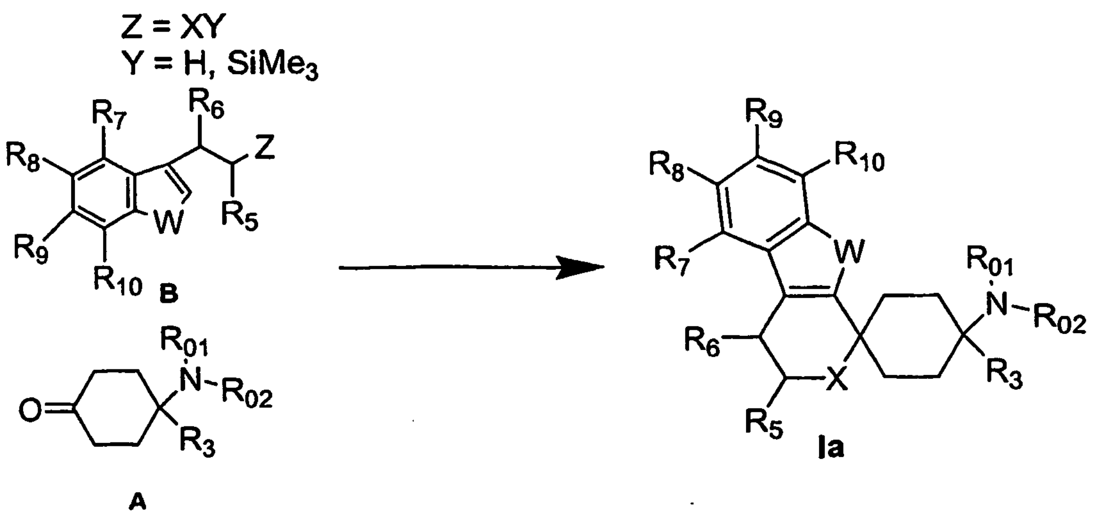

Zur Herstellung der Verbindungen der allgemeinen Formel Ia werden Ketone der allgemeinen Formel A mit Heteroaromaten der allgemeinen Formel B unter Zugabe von Säure oder deren Trimethylsilylester, beispielsweise Trifluormethansulfonsäuretrimethylsilylester, Essigsäure, Phosphorsäure, Methansulfonsäure oder Trifluoressigsäure in einem geeigneten Lösungsmittel, beispielsweise Dichlorethan, Dichlormethan, Chloroform, Acetonitril, Diethylether oder Nitromethan, umgesetzt.

Die Herstellung geeigneter 4-Aminocyclohexanone ist aus der Literatur bekannt (Lednicer et al., J. Med. Chem., 23, 1980, 424-430; WO 0290317; US 4065573).

Alternativ kann die Herstellung auch nach folgendem Schema erfolgen, wobei X, W, $R^3$, $R^5$, $R^6$, $R^7$, $R^8$, $R^9$ und $R^{10}$ die für erfindungsgemäße Verbindungen gemäß Formel I angegebene Bedeutung haben,
und
$R^{01}$ und $R^{02}$ die für erfindungsgemäße Verbindungen gemäß Formel I für $R^1$ und $R^2$ angegebene Bedeutung haben und zusätzlich unabhängig voneinander für eine Schutzgruppe stehen können.

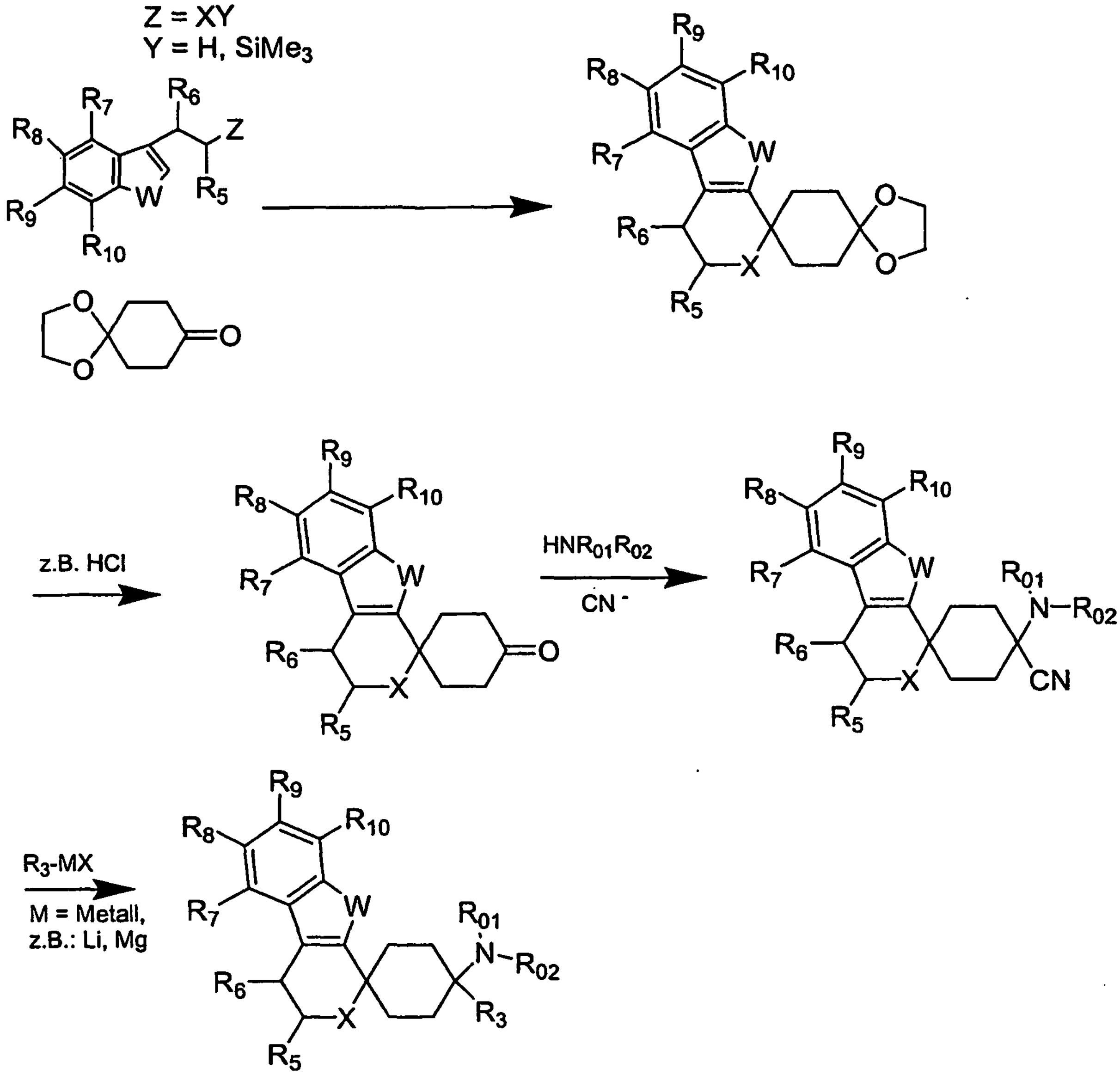


Spirocyclische Cyclohexanderivate der allgemeinen Formel I, bei denen X $NR^{17}$ und $R^{17}$ $COR^{12}$ oder $SO_2R^{12}$ bedeutet, können durch die Umsetzung von spirocyclischen Cyclohexanderivaten der allgemeinen Formel I, bei denen X NH bedeutet, durch Umsetzung mit einem Anhydrid oder einem Säurechlorid unter Zugabe einer Base, beispielsweise Triethylamin, erhalten werden. Vorzugsweise findet diese Reaktion unter Mikrowelleneinstrahlung statt.

Spirocyclische Cyclohexanderivate der allgemeinen Formel I, bei denen X SO oder $SO_2$ bedeuten, können

durch Umsetzung von spirocyclischen Cyclohexanderivaten der allgemeinen Formel I, bei denen X S bedeuten, mit einem Oxidationsmittel, beispielsweise $H_2O_2$, erhalten werden.

Spirozyklische Cyclohexanderivaten, bei denen $R^3$ für 3-Thienyl und, $R^1$ für $CH_3$ und $R^2$ für H steht, können nach der folgenden Beschreibung hergestellt werden, wobei R' und R" unabhängig voneinander für eine Schutzgruppe stehen:

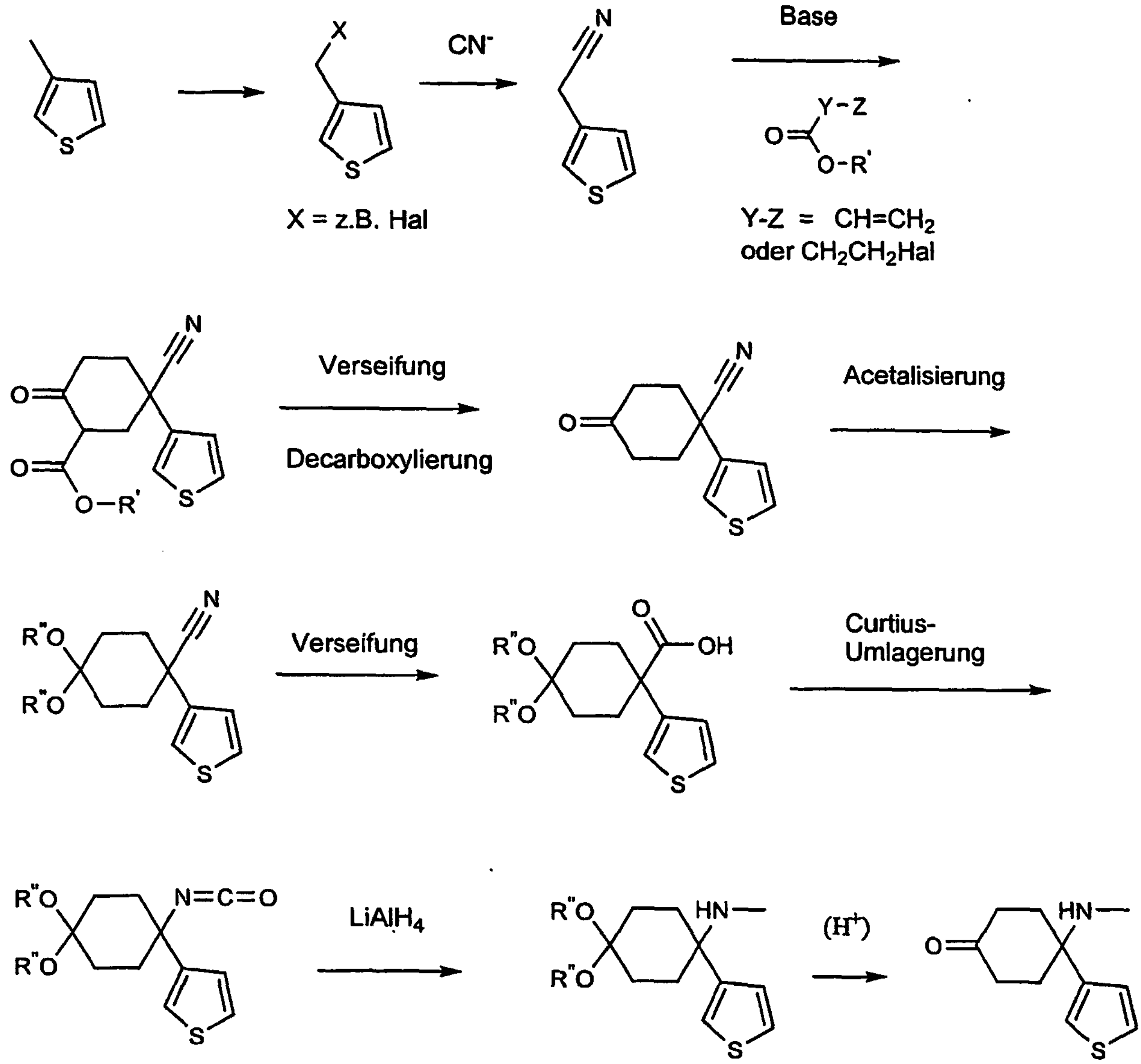

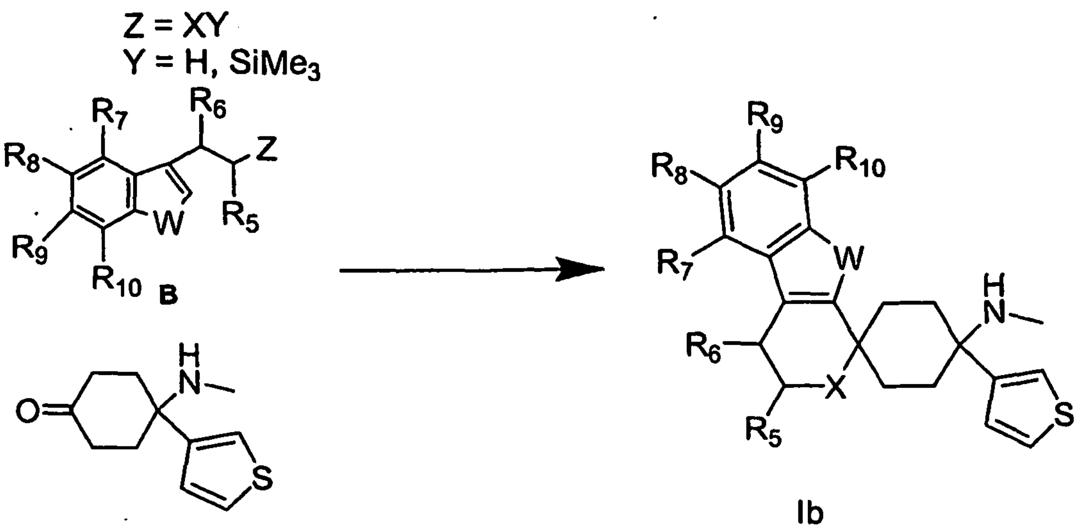


B

Ib

In diesem Verfahren wird in die Methylgruppe des 3-Methylthiophens nach dem Fachmann bekannten Methoden, z.B. durch Bromierung mit N-Bromsuccinimid in einem inerten Lösungsmittel wie z.B. Benzol, unter Zusatz eines Initiators wie z.B. Benzoylperoxid und ggf. unter Erwärmung, eine Abgangsgruppe, wie z.B. ein Halogen, vorzugsweise Brom, eingeführt.

Das so erhaltene Produkt, z.B. 3-Brommethyl-thiophen, wird unter Einsatz einer Cyanidquelle, wie z.B. Natriumcyanid, z.B. in Gegenwart eines quarternären Ammoniumsalzes wie z.B. Tetrabutylammoniumbromid, ggf. unter Erwärmung, in das entsprechende Nitril überführt.

Das so erhaltene Thiophen-3-yl-acetonitril wird in Gegenwart von einem Acrylester oder einem 3-Brompropionsäureester im Überschuß, vorzugsweise mit 2,3 Mol-Äquivalenten 3-Brompropionsäureethylester, sowie in Gegenwart einer Base, z.B. Natriumamid, in einem aprotischen Lösungsmittel, z.B. Toluol, umgesetzt, wobei ggf. erwärmt werden kann.

Die so erhaltenen 5-Cyano-2-oxo-5-thiophen-3-yl-cyclohexancarbonsäureester können nach dem Fachmann geläufigen Verfahren verseift und decarboxyliert werden, vorzugsweise durch Erhitzen in einer Mischung aus konzentrierter Salzsäure und Eisessig unter Rückfluß.

Die Ketogruppe des so erhaltenen 4-Oxo-1-thiophen-3-yl-cyclohexancarbonitrils kann nach dem Fachmann bekannten Verfahren mit einer Schutzgruppe versehen werden, vorzugsweise durch Acetalisierung, besonders bevorzugt durch Umwandlung in die Ethylendioxyschutzgruppe, ganz besonders bevorzugt durch Erwärmen des Ketons in Toluol in Gegenwart von Ethylenglykol und von einem sauren Katalysator, z.B. para-Toluolsulfonsäure, unter Erwärmen, vorzugsweise unter Rückfluß.

Das so erhaltene 8-Thiophen-3-yl-1,4-dioxa-spiro[4.5]decan-8-carbonitril kann nach dem Fachmann bekannten Verfahren durch Verseifung der Nitrilgruppe in die entsprechende Carbonsäure überführt werden, z.B. im basischem Medium, vorzugsweise mit Natriumhydroxid in Ethylenglykol unter Rückfluß.
Die so erhaltene 8-Thiophen-3-yl-1,4-dioxa-spiro[4.5]decan-8-carbonsäure kann nach dem Fachmann bekannten Verfahren in das entsprechende Isocyanat überführt werden, vorzugsweise durch Umsetzungen, die nach Art einer Curtius-Umlagerung verlaufen. Bevorzugt wird dabei die Carbonsäure mit Azidophosphorsäurediphenylester in Gegenwart von Triethylamin in Anisol, unter Erwärmen zum Rückfluß, in das Isocyanat überführt.

Das so erhaltene 8-Isocyanato-8-thiophen-3-yl-1,4-dioxa-spiro[4.5]decan kann z.B. mit Lithiumaluminiumhydrid in einem aprotischen Lösungsmittel, vorzugsweise Tetrahydrofuran, in die entsprechende Methylamino-Verbindung überführt werden. Das so erhaltene Methyl-(8-thiophen-3-yl-1,4-dioxa-spiro[4.5]dec-8-yl)-amin kann säurekatalysiert zum 4-Methylamino-4-thiophen-3-yl-cyclohexanon entschützt und anschließend z.B. mit Verbindungen der allgemeinen Formel B zu spirocyclischen Cyclohexanderivaten umgesetzt werden.

## Beispiele

Die folgenden Beispiele dienen zur näheren Erläuterung der Erfindung, schränken aber den allgemeinen Erfindungsgedanken nicht ein.

Die Ausbeuten der hergestellten Verbindungen sind nicht optimiert.

Alle Temperaturen sind unkorrigiert.

Die Angabe "Ether" bedeutet Diethylether, "EE" Ethylacetat, "DCM" Dichlormethan, "DMF" Dimethylformamid,

"DMSO" Dimethylsulfoxid und "THF" Tetrahydrofuran. Die Angabe "Äquivalente" bedeutet Stoffmengenäquivalente, "Smp." Schmelzpunkt bzw. Schmelzbereich, "Zers." Zersetzung, "RT" Raumtemperatur , "abs." absolut (wasserfrei), ,"rac." racemisch , "konz." konzentriert, "min" Minuten, "h" Stunden, "d" Tage, "Vol.%" Volumenprozent, "m%" Massenprozent und "M" ist eine Konzentrationsangabe in molll.

Als stationäre Phase für die Säulenchromatographie wurde Kieselgel 60 (0.040 - 0.063 mm) der Firma E. Merck, Darmstadt, eingesetzt.

Die dünnschicht-chromatographischen Untersuchungen wurden mit HPTLC-Fertigplatten, Kieselgel 60 F 254, der Firma E. Merck, Darmstadt, durchgeführt.

Die Mischungsverhältnisse von Laufmitteln für chromatographische Untersuchungen sind stets in Volumen/Volumen angegeben.

## Beispiele

**Beispiel 1:** 1,1-[3-Dimethylamino-3-(pyridin-2-yl)pentamethylen]-3,4-dihydro-1H-2,9-diazafluoren, Diastereoisomerengemisch

4-Dimethylamino-4-pyridin-2-ylcyclohexanon (4,37 g, 20 mmol) und 2-(1H-Indol-3-yl)-ethylamin ("Tryptamin ", 3,2 g, 20 mmol) wurden unter Argon in trockenem MeOH (200 ml) gelöst. Nach einer Reaktionszeit von 24 h wurde MeOH abdestilliert, der gelbe, ölige Rückstand in 1,2-Dichlorethan (200 ml) suspendiert, Trifluoressigsäure (20 ml) zugegeben und 2 h bei RT gerührt. Zur Aufarbeitung wurde der Ansatz mit Wasser (100 ml) verdünnt und mit NaOH (5 mol/l) auf pH 11 eingestellt. Nach Zugabe von EE (50 ml) fiel beim Rühren ein weißer Feststoff aus, der über eine Fritte abgesaugt wurde. Der Feststoff wurde mit Wasser (3 × 25 ml) gewaschen und im Vakuum getrocknet. 1,1-(3-Dimethylamino-3-(pyridin-2-yl)pentamethylen)-3,4-dihydro-1 H-2,9-diazafluoren wurde als Diastereoisomerengemisch erhalten (4,9 g weißer Feststoff, Smp. 122-125 °C).

**Beispiel 2:** 2-Acetyl-1,1-[3-dimethylamino-3-(pyridin-2-yl)pentamethylen]-3,4-dihydro-1 H-2,9-diazafluoren

Das nach Beispiel 1 erhaltene 1,1-(3-Dimethylamino-3-(pyridin-2-yl)pentamethylen)-3,4-dihydro-1H-2,9-diazafluoren (200 mg, 0,56 mmol) wurde in Pyridin (5 ml) gelöst, Acetanhydrid (484 μl, 5,6 mmol) zugetropft und 5 d bei RT gerührt. Zur Aufarbeitung wurde Pyridin am Rotationsverdampfer abdestilliert, der Rückstand mit Wasser (10 ml) verdünnt, mit 5M NaOH auf pH 11 eingestellt und mit EE (3 × 10 ml) extrahiert. Aus den vereinigten organischen Extrakten fiel ein Feststoff aus, der abgesaugt und getrocknet wurde. Es wurden 160 mg eines diastereoisomerenreinen weißen Feststoffs erhalten. 150 mg (0,37 mmol) hiervon wurden in heißem Ethanol (10 ml) gelöst und mit einer ebenfalls heißen Lösung von Citronensäure (72 mg, 0,37 mmol) in Ethanol (1 ml) versetzt. Nach dem Abkühlen auf ca. 5 °C wurde der Ansatz 4 h stehen gelassen, dann zur Trockene eingeengt. Das Citrat von 2-Acetyl-1,1-(3-dimethylamino-3-(pyridin-2-yl)pentamethylen)-3,4-dihydro-1H-2,9-diazafluoren wurde so in einer Ausbeute von 222 mg erhalten (weißer Schaum, Smp. 108-112 °C).

**Beispiel 3:** 1,1-[3-Dimethylamino-3-(pyridin-2-yl)pentamethylen]-3,4-dihydro-1H-2-oxa-9-thiafluoren Citrat

Unter Argon wurden 4-Dimethylamino-4-pyridin-2-ylcyclohexanon (218 mg, 1 mmol) und 2-Benzo[b]thiophen-2-ylethanol (178 mg, 1 mmol) in abs. DCM (5 ml) gelöst, Methansulfonsäure (3 ml) zugegeben und der Ansatz 3 d bei RT gerührt. Zur Aufarbeitung wurde das Reaktionsgemisch mit Eis (5 g) und Wasser (30 ml) versetzt. Nach der Neutralisation mit Natriumhydrogencarbonat (4,4 g, 52 mmol) und der Zugabe von 5M NaOH (1 ml) wurde DCM (10 ml) hinzugefügt, die organische Phase abgetrennt und die wäßrige Phase mit DCM (2 × 30 ml) extrahiert. Die vereinigten organischen Phasen wurden nach dem Trocknen eingeengt und der Rückstand (375 mg) chromatographisch an Kieselgel (45 g, Eluent: EE/Methanol 10 : 1 gefolgt von 4 : 1, dann Methanol) getrennt. Das Rohprodukt wurde in einer Ausbeute von 143 mg (0,377 mmol) als weißer Feststoff erhalten (Smp. 155-168 °C), in Ethanol (10 ml) bei 50 °C gelöst, mit Citronensäure (72 mg, 0,377 mmol), gelöst in warmem Ethanol (3 ml), versetzt, 2 h bei RT gerührt und auf 5 ml eingeengt. Der dabei ausgefallene Feststoff wurde abgesaugt und mit Ethanol (2 × 1 ml) gewaschen. Das Citrat von 1,1-[3-Dimethylamino-3-(pyridin-2-yl)pentamethylen]-3,4-dihydro-1H-2-oxa-9-thiafluoren wurde in einer Ausbeute von 179 mg erhalten (weißer Feststoff, Smp. 189-191 °C).

**Beispiel 4:** 1,1-[3-Dimethylamino-3-(pyridin-2-yl)pentamethylen]-1,3,4,9-tetrahydropyrano[3,4-b]indol Hemicitrat, unpolareres Diastereoisomer

4-Dimethylamino-4-pyridin-2-ylcyclohexanon (654 mg, 3 mmol) und 2-(1 H-Indol-3-yl)ethanol ("Tryptophol", 483 mg, 3 mmol) wurden in DCM (50 ml) vorgelegt, innerhalb von 3 min Methansulfonsäure (400 μl, 6,2 mmol) zugegeben

und 70 h bei RT gerührt. Zur Aufarbeitung wurde das Reaktionsgemisch mit 2M NaOH (15 ml) versetzt, 20 min gerührt, die organische Phase abgetrennt und die verbliebende wäßrige Phase mit Dichlormethan (3 × 20 ml) ausgeschüttelt. Die vereinigten organischen Phasen wurden mit Wasser (2 × 30 ml) gewaschen; getrocknet, filtriert und eingeengt. Der erhaltene Rückstand wurde an Kieselgel (60 g, EE/Ethanol 2 : 1) chromatographiert und die Base des unpolareren Diastereoisomeren des Zielprodukts in einer Ausbeute von 123 mg gewonnen. 108 mg (0,3 mmol) hiervon wurden heißem Ethanol (15 ml) gelöst, mit einer ebenfalls heißen ethanolischen Citronensäurelösung (58 mg, 0,3 mmol in 1 ml) versetzt und der Ansatz 12 h bei 5 °C belassen. Der entstandene Feststoff wurde abgesaugt. Das Hemicitrat des unpolareren Diastereoisomeren von 1,1-[3-Dimethylamino-3-(pyridin-2-yl)pentamethylen]-1,3,4,9-tetrahydropyrano [3,4-b]indol wurde so in einer Ausbeute von 79 mg erhalten (weißer Feststoff, Smp. 255-260 °C)

**Beispiel 5:** 1,1-[3-Dimethylamino-3-(pyridin-2-yl)pentamethylen]-1,3,4,9-tetrahydropyrano[3,4-b]indol Citrat, polareres Diastereoisomer

Wie für Beispiel 4 beschrieben wurden auch 415 mg des polareren Diastereoisomeren von 1,1-[3-Dimethylamino-3-(pyridin-2-yl)pentamethylen]-1,3,4,9-tetrahydropyrano[3,4-b]indol erhalten. 400 mg (1,1 mmol) hiervon wurden in heißem Ethanol (12 ml) gelöst und heiße ethanolische Citronensäurelösung (insgesamt 211 mg, 1,1 mmol in 2 ml) zugegeben. Der Ansatz wurde 2 h bei 5 °C belassen und dann zur Trockene eingeengt. Das Citrat des polareren Diastereoisomeren von 1,1-[3-Dimethylamino-3-(pyridin-2-yl)pentamethylen]-1,3,4,9-tetrahydropyrano[3,4-b]indol wurde so in einer Ausbeute von 612 mg erhalten (weißer glasartiger Feststoff, Smp. 96-100 °C).

**Beispiel 6** 1,1-[3-Dimethylamino-3-(2-thienyl)pentamethylen]-1,3,4,9-tetra-hydro-pyrano[3,4-b]indol Dimethansulfonat und **Beispiel 7** 1,1-[3-Dimethylamino-3-(2-thienyl)pentamethylen]-1,3,4,9-tetra-hydropyrano[3,4-b]indol Citrat

4-Dimethylamino-4-(2-thienyl)-cyclohexanon (223 mg, 1 mmol) und 2-(1 H-Indol-3-yl)ethanol (161 mg, 1 mmol) wurden in abs. DCM gelöst und Methansulfonsäure (0,071 ml, 1,1 mmol) zugegeben. Es wurde 16 h bei RT gerührt, wobei das polarere Diastereomer von 1,1-[3-Dimethylamino-3-(2-thienyl)pentamethylen]-1,3,4,9-tetra-hydro-pyrano [3,4-b]indol als Dimethansulfonat ausfiel (Beispiel 6). Man erhielt den hellgrauen Feststoff in einer Ausbeute von 25 % (117 mg; Smp. 132°C).

Das Filtrat wurde mit 1 M NaOH (20 ml) versetzt und 16 h bei RT gerührt. Die organische Phase wurde abgetrennt, die wässrige Phase mit DCM extrahiert und die vereinigten organischen Phasen wurden eingeengt. Dabei wurde ein Substanzgemisch erhalten, das chromatographisch aufgetrennt wurde [Kieselgel G (20 g); EE/Methanol 8: 1]. Das unpolarere Diastereoisomer von 1,1-[3-Dimethylamino-3-(2-thienyl)pentamethylen]-1,3,4,9-tetra-hydro-pyrano [3,4-b]indol wurde dabei in einer Ausbeute von 54 % (196 mg, Smp. 235-238 °C), das polarere Diastereoisomer in einer Ausbeute von 10 % (38 mg) gewonnen.

Zur Herstellung des Citrates wurde das unpolarere Diastereoisomer von 1,1-[3-Dimethylamino-3-(2-thienyl) pentamethylen]-1,3,4,9-tetra-hydro-pyrano[3,4-b]indol (170 mg, 0,46 mmol) unter Erwärmen in Ethanol (50 ml) gelöst und mit Citronensäure (98 mg, 0,51 mmol) in Ethanol (5 ml) versetzt. Es wurde 1 h bei RT gerührt. Das Citrat (Beispiel 7) fiel als farblose Verbindung in einer Ausbeute von 60% (153 mg; Smp. 222-225°C) an.

**Beispiel 8** 1,1-[3-Dimethylamino-3-(3-thienyl)pentamethylen]-1,3,4,9-tetra-hydropyrano[3,4-b]indol Hemicitrat und **Beispiel 9** 1,1-[3-Dimethylamino-3-(3-thienyl)pentamethylen]-1,3,4,9-tetra-hydropyrano[3,4-b]indol Citrat

Das 4-Dimethylamino-4-(3-thienyl)-cyclohexanon (223 mg, 1 mmol) und 2-(1 H-Indol-3-yl)ethanol (161 mg, 1 mmol) wurden in abs. DCM (50 ml) gelöst und mit Methansulfonsäure (0,13 ml, 2,0 mmol) versetzt. Der Ansatz wurde 2 d bei RT gerührt, wobei ein Teil des polareren Diastereoisomers von 1,1-[3-Dimethylamino-3-(3-thienyl)pentamethylen]-1,3,4,9-tetra-hydro-pyrano[3,4-b]indol als Methansulfonat ausfiel. Der Feststoff wurde abgesaugt, mit DCM gewaschen und in einer Ausbeute von 12 % (55 mg) gewonnen. Das Filtrat wurde mit 0,5M NaOH (20 ml) versetzt und 2 h bei RT gerührt. Dabei fiel das unpolarere Diastereoisomer von 1,1-[3-Dimethylamino-3-(3-thienyl)pentamethylen]- 1,3,4,9-tetra-hydro-pyrano[3,4-b]indol als farbloser Feststoff aus und wurde nach Filtration in einer Ausbeute von 38% (138 mg) mit einem Smp von 291-294 °C erhalten. Die organische Phase des Filtrats wurde abgetrennt und die wässrige Phase mit DCM (2 × 20 ml) extrahiert. Die vereinigten organischen Phasen lieferten ein Diastereoisomerengemisch (184 mg, 50 %). Nach Versetzen mit Methanol (10 ml) wurde nur das polarere Diastereoisomer von 1,1-[3-Dimethylamino-3-(3-thienyl)pentamethylen]-1,3,4,9-tetra-hydro-pyrano[3,4-b]indol (45 mg, 12 %, Fp. 235-238 °C) gelöst, der Rückstand war das unpolarere Diastereoisomer.

Zur Herstellung des Citrates wurde das unpolarere Diastereoisomer von 1,1-[3-Dimethylamino-3-(3-thienyl)pentamethylen]-1,3,4,9-tetra-hydro-pyrano[3,4-b]indol (111 mg, 0,3 mmol) in Ethanol (35 ml) unter Erwärmen bei 50 °C suspendiert und mit Citronensäure (60 mg, 0,31 mmol) in Ethanol (5 ml) versetzt. Es wurde 16 h bei RT gerührt. Das ausgefallene Hemicitrat (Beispiel 8) wurde abgesaugt und mit Ethanol (2 × 5 ml) gewaschen. Die farblose Verbindung wurde in einer

Ausbeute von 79 % (110 mg; Smp. 246-250°C) erhalten.
Das polarere Diastereoisomer (81 mg, 0,22 mmol) wurde in Ethanol (20 ml) gelöst, mit Citronensäure (46 mg, 0,24 mmol) in Ethanol (3 ml) versetzt und 16 h bei RT gerührt. Die klare Mischung wurde auf 3 ml eingeengt, mit Diethylether (40 ml) versetzt und 15 min bei Raumtemperatur gerührt. Das polarere Citrat fiel als farbloser Feststoff in einer Ausbeute von 63 % (77 mg; Smp. 245-248°C) aus.

**Beispiel 10** 1,1-[3-Dimethylamino-3-(2-thienyl)pentamethylen]-1,3,4,9-tetra-hydropyrano[3,4-b]-6-fluorindol Hemicitrat und **Beispiel 11** 1,1-[3-Dimethylamino-3-(2-thienyl)pentamethylen]-1,3,4,9-tetra-hydropyrano[3,4-b]-6-fluorindol Citrat

4-Dimethylamino-4-(2-thienyl)-cyclohexanon (223 mg, 1 mmol) und 5-Fluor-2-(1 H-indol-3-yl)ethanol (179 mg, 1 mmol) wurden in abs. DCM (50 ml) vorgelegt und mit Methansulfonsäure (0,13 ml, 2,0 mmol) versetzt. Der Ansatz wurde 20 h bei RT gerührt und anschließend mit 0,5M NaOH (20 ml) versetzt und 2 h bei RT gerührt. Die organische Phase wurde abgetrennt und die wässrige Phase mit DCM extrahiert. Aus den organischen Phasen wurde ein Diastereoisomerengemisch von 1,1-[3-Dimethylamino-3-(2-thienyl)pentamethylen]-1,3,4,9-tetra-hydro-pyrano[3,4-b]-6-fluorindol (382 mg) erhalten. Dieses wurde aus Propan-2-ol (70 ml) umkristallisiert. Dabei fiel das unpolarere Diastereoisomer aus (165 mg, 43%). Aus dem Filtrat wurde nach dem Einengen ein Diastereoisomerengemisch isoliert (211 mg). Nach chromatographischer Trennung dieses Gemisches [Kieselgel G (40 g); EE/Cyclohexan 1:1 (400 ml), EE (400 ml), EE/Methanol 4:1 (300 ml)] wurden das unpolarere Diastereoisomer (67 mg, 17 %, Smp. 225-230 °C) und das polarere Diastereoisomer (110 mg, 29 %, Smp. 197-202 °C) als farblose Feststoffe gewonnen.
Zur Herstellung des Citrates wurde das unpolarere Diastereoisomer (165 mg, 0,43 mmol) in Ethanol (50 ml) unter Erwärmen suspendiert und mit Citronensäure (93 mg, 0,48 mmol) in Ethanol (5 ml) versetzt. Es wurde 30 min bei 50°C und 16 h bei RT gerührt. Das Hemicitrat wurde abgesaugt und mit Ethanol gewaschen. Die farblose Verbindung wurde in einer Ausbeute von 54 % (111 mg; Smp. 199-201°C) erhalten (Beispiel 10).
Das polarere Diastereoisomer (91 mg, 0,236 mmol) wurde in Ethanol (15 ml) bei 40 °C gelöst, mit Citronensäure (52 mg, 0,27 mmol) in Ethanol (5 ml) versetzt und 2 h bei RT gerührt. Die Lösung wurde auf 3 ml eingeengt, mit Ether (40 ml) versetzt und 16 h bei RT gerührt. Das polarere Hemicitrat fiel als farbloser Feststoff in einer Ausbeute von 93 % (106 mg; Smp. 137-140°C) aus (Beispiel 11).

**Beispiel 12** 1,1-[3-Dimethylamino-3-(3-thienyl)pentamethylen]-1,3,4,9-tetra-hydropyrano[3,4-b]-6-fluorindol Dimethansulfonat und **Beispiel 13** 1,1-[3-Dimethylamino-3-(3-thienyl)pentamethylen]-1,3,4,9-tetra-hydropyrano[3,4-b]-6-fluorindol Hemicitrat

4-Dimethylamino-4-(3-thienyl)-cyclohexanon (446,6 mg, 2 mmol) und 5-Fluor-2-(1 H-indol-3-yl)ethanol (394,4 mg, 2 mmol) wurden in abs. 1,2-Dichlorethan (30 ml) gelöst und mit Methansulfonsäure (0,13 ml, 2 mmol) versetzt. Der Ansatz wurde 20 h bei RT gerührt. Dann wurde das ausgefallene Methansulfonat des polareren Diastereoisomers abgesaugt und mit 1,2-Dichlorethan gewaschen. Der hellgraue Feststoff wurde in einer Ausbeute von 76 % (733 mg; Smp. 143-145°C) gewonnen (Beispiel 12).
Das Filtrat wurde mit 1 M NaOH (30 ml) versetzt und 2 h bei RT gerührt. Dabei fiel das unpolarere Diastereoisomer als farbloser Feststoff aus und wurde in einer Ausbeute von 8 % (58,5 mg) erhalten. Die Phasen des Filtrats wurden getrennt und die wässrige Phase mit DCM extrahiert. Die vereinigten organischen Phasen enthielten ein Diastereoisomerengemisch (300,3 mg).
Zur Herstellung des Citrats wurde das Diastereoisomerengemisch (126 mg, 0,33 mmol) unter Erwärmen bei 50°C in Ethanol (100 ml) suspendiert und mit Citronensäure (69,2 mg, 0,36 mmol) in Ethanol (5 ml) versetzt. Es wurde 2 h bei RT gerührt und über Nacht bei 10 °C aufbewahrt. Das ausgefallene Hemicitrat des unpolareren Diastereoisomers wurde abgesaugt. Die farblose Verbindung wurde in einer Ausbeute von 60 % (94 mg; Smp. 227-229°C) erhalten (Beispiel 13).

**Beispiel 14** 1,1-[3-Methylamino-3-(2-thienyl)pentamethylen]-1,3,4,9-tetrahydropyrano[3,4-b]indol Citrat

4-Methylamino-4-thiophen-2-yl-cyclohexanon (418,6 mg, 2,0 mmol) und 2-(1 H-Indol-3-yl)-ethanol (322,4 mg, 2,0 mmol) wurden in 50ml DCM gelöst und schnell mit Trifluormethansulfonsäure (0,18 ml, 2,03 mmol) versetzt. Nach 20h Rühren bei RT wurde der Ansatz 20min. mit 20ml 2M NaOH gerührt. Die organische Phase wurde abgetrennt und die wässrige Phase mit DCM extrahiert. Die vereinigten organischen Phasen wurden im Vakuum zur Trockene eingeengt und der Rückstand in 25ml Methanol suspendiert. Der farblose Feststoff wurde abgesaugt, und man erhielt so in einer Ausbeute von 363mg (51%) 1,1-[3-Methylamino-3-(2-thienyl)pentamethylen]-1,3,4,9-tetrahydro-pyrano[3,4-b]indol.
Zur Herstellung des Citrats wurde 1,1-[3-Methylamino-3-(2-thienyl)pentamethylen]-1,3,4,9-tetrahydro-pyrano[3,4-b]indol (352 mg, 1,0 mmol) in heißem Ethanol (30 ml) gelöst und mit Citronensäure (200 mg, 1,04 mmol) in heißem Ethanol (5 ml) versetzt. Es wurde 15h bei 5°C stehen gelassen. Das ausgefallene Citrat wurde abgesaugt und als farblose Verbindung in einer Ausbeute von 69% (377mg; Smp. 201-203°C) erhalten (Beispiel 14).

**Beispiel 15** 1,1-[3-Methylamino-3-(2-thienyl)pentamethylen]-1,3,4,9-tetrahydropyrano[3,4-b]-6-fluorindol Citrat

4-Methylamino-4-thiophen-2-yl-cyclohexanon (418,6 mg, 2,0 mmol) und 2-(5-Fluor-1 H-indol-3-yl)-ethanol (358,3 mg, 2,0 mmol) wurden in 50ml DCM gelöst und schnell mit Trifluormethansulfonsäure (0,18 ml, 2,03 mmol) versetzt. Nach 20h Rühren bei RT wurde der Ansatz 20min. mit 20ml 2M NaOH gerührt. Die organische Phase wurde abgetrennt und die wässrige Phase mit DCM extrahiert. Die vereinigten organischen Phasen wurden im Vakuum zur Trockene eingeengt und der Rückstand in Methanol suspendiert. Der farblose Feststoff wurde abgesaugt, und man erhielt so in einer Ausbeute von 697mg (94%) 1,1-[3-Methylamino-3-(2-thienyl)pentamethylen]-1,3,4,9-tetrahydro-pyrano[3,4-b]-6-fluorindol.
Zur Herstellung des Citrats wurde der spirocyclische Ether (680 mg, 1,84 mmol) in heißem Ethanol (50 ml) gelöst und mit Citronensäure (384 mg, 2,0 mmol) in heißem Ethanol (10 ml) versetzt. Es wurde 15h bei 5°C stehen gelassen. Das ausgefallene Citrat wurde abgesaugt und als farblose Verbindung in einer Ausbeute von 67% (694mg; Smp. 207-209°C) erhalten (Beispiel 15).

**3-Brommethyl-thiophen**

N-Bromsuccinimid (35,6 g; 0,20 mol) und Benzoylperoxid (0,4 g; 0,0013 mol) wurden binnen 90 min bei 90 °C portionsweise zu einer Mischung von 3-Methylthiophen (22 g; 0,203 mol) und Benzoylperoxid (0,4 g; 0,0013 mol) in trockenem Benzol gegeben. Nach vollständiger Umsetzung (dünnschichtchromatographische Reaktionskontrolle) wurde auf 0°C abgekühlt und gefiltert. Das Filtrat wurde im Vakuum eingeengt. Es wurden 34 g 3-Brommethyl-thiophen (rotbraune Flüssigkeit) erhalten.

**Thiophen-3-yl-acetonitril**

Natriumcyanid (12,03 g; 0,25 mol) und katalytische Mengen Tetra-n-butylammoniumbromid wurden zu einer Mischung von 3-Brommethyl-thiophen (29 g; 0,16 mol) in Dichlormethan (175 ml) und Wasser (50 ml) gegeben. Die Reaktionsmsichung wurde unter Rückfluß gerührt. Nach vollständiger Umsetzung (dünnschichtchromatographische Reaktionskontrolle) wurde die organische Phase abgetrennt und mit Wasser gewaschen (3 × 500 ml), getrocknet (Natriumsulfat) und im Vakuum eingeengt. Säulenchromatographische Reinigung (Kieselgel, 3% Essigester in n-Hexan) ergab 9 g Thiophen-3-yl-acetonitril (44 %; rotbraune Flüssigkeit).

**5-Cyano-2-oxo-5-thiophen-3-yl-cyclohexancarbonsäureethylester**

Zu Thiophen-3-yl-acetonitril (27,5 g; 0,22 mol) gelöst in 350 ml Toluol wurden 3-Brompropionsäureethylester (96,14 g; 0,53 mol) zugefügt. Anschließend wurde Natriumamid (74,03 g; 1,9 mol) portionsweise binnen 1 h bei 0 bis 10 °C zugefügt. Die Reaktionsmischung wurde danach ca. 1 h unter Rückfluß gerührt. Nach vollständiger Umsetzung (dünnschichtchromatographische Reaktionskontrolle) wurde überschüssiges Natriumamid mit Essigsäure/ Wasser (500 ml; 2 : 1) bei 0 bis 5 °C zerstört. Die organische Phase wurde abgetrennt und mit Natriumhydrogencarbonatlösung (300 ml) neutralisiert, getrocknet (Natriumsulfat) und im Vakuum eingeengt. Es wurden 40 g 5-Cyano-2-oxo-5-thiophen-3-yl-cyclohexancarbonsäureethylester (gelbe Flüssigkeit) erhalten.

**4-Oxo-1-thiophen-3-yl-cyclohexancarbonitril**

5-Cyano-2-oxo-5-thiophen-3-yl-cyclohexancarbonsäureethylester (40 g; 0,14 mol) gelöst in einer Mischung aus konzentrierter Salzsäure (200 ml) und Eisessig (400 ml) wurde unter Rühren ca. 4 h zum Rückfluß erhitzt. Nach vollständiger Umsetzung (dünnschichtchromatographische Reaktionskontrolle) wurde Wasser (100 ml) hinzugefügt und mit wäßriger Natronlauge (200 ml) neutralisiert sowie mit Essigester (2 × 400 ml) extrahiert. Die organische Phase wurde mit Natriumhydrogencarbonatlösung (200 ml) und Wasser (100 ml) gewaschen, getrocknet (Natriumsulfat) und im Vakuum eingeengt. Säulenchromatographische Reinigung (Kieselgel, 25% Essigester in n-Hexan) ergab 12,5 g 4-Oxo-1-thiophen-3-yl-cyclohexancarbonitril (42%; blaßgelber Feststoff).

**8-Thiophen-3-yl-1,4-dioxa-spiro[4.5]decan-8-carbonitril**

Zu 4-Oxo-1-thiophen-3-yl-cyclohexancarbonitril (22 g; 0,107 mol) gelöst in Toluol (500 ml) wurden katalytische Mengen para-Toluolsulfonsäure und Ethylenglykol (13,3 g; 0,21 mol) gegeben. Die Reaktionsmischung wurde ca. 2 h unter Rückfluß gerührt. Nach vollständiger Umsetzung (dünnschichtchromatographische Reaktionskontrolle) wurde die Toluolphase abgetrennt, mit Natriumhydrogencarbonatlösung (200 ml) gewaschen, getrocknet (Natriumsulfat) und im Vakuum eingeengt. Es wurden 25 g 8-Thiophen-3-yl-1,4-dioxaspiro[4.5]decan-8-carbonitril (95%; farbloser Feststoff)

erhalten.

**8-Thiophen-3-yl-1,4-dioxa-spiro[4.5]decan-8-carbonsäure**

Zu 8-Thiophen-3-yl-1,4-dioxa-spiro[4.5]decan-8-carbonitril (25 g; 0,095 mol) gelöst in Ethylenglykol (226 ml) wurde Kaliumhydroxid (28 g; 0,5 mol) gegeben. Die Reaktionsmischung wurde ca. 12 h unter Rückfluß gerührt. Nach vollständiger Umsetzung (dünnschichtchromatographische Reaktionskontrolle) wurde die Reaktionsmischung mit verdünnter Salzsäure auf pH ca. 1 eingestellt. Der entstandene Niederschlag wurde gefiltert und getrocknet. Es wurden 15 g 8-Thiophen-3-yl-1,4-dioxa-spiro[4.5]decan-8-carbonsäure (55 %; blaßgelber Feststoff) erhalten.

**8-Isocyanato-8-thiophen-3-yl-1,4-dioxa-spiro[4.5]decan**

Zu 8-Thiophen-3-yl-1,4-dioxa-spiro[4.5]decan-8-carbonsäure (15 g; 56 mmol) gelöst in Anisol (160 ml) wurden Azidophosphorsäurediphenylester (15,4 g; 56 mmol) und Triethylamin (5,66 g; 55 mmol) gegeben. Die Reaktionsmischung wurde 2 h auf 90 bis 100 °C erhitzt. Nach vollständiger Umsetzung (dünnschichtchromatographische Reaktionskontrolle) wurde säulenchromatographisch gereinigt (Kieselgel, 10% Essigester in n-Hexan). Es wurden 6 g 8-Isocyanato-8-thiophen-3-yl-1,4-dioxaspiro[4.5]decan erhalten (41%; farblose Flüssigkeit).

**Methyl-(8-thiophen-3-yl-1,4-dioxa-spiro[4.5]dec-8-yl)-amin**

Zu 8-Isocyanato-8-thiophen-3-yl-1,4-dioxa-spiro[4.5]decan (6 g; 22,6 mmol) gelöst in trockenem THF (70 ml) wurde portionsweise Lithiumaluminiumhydrid (1,7 g) bei 0 bis 5 °C gegeben. Die Reaktionsmischung wurde ca. 1,5 h unter Rückfluß gerührt. Nach vollständiger Umsetzung (dünnschichtchromatographische Reaktionskontrolle) wurde überschüssiges Lithiumaluminiumhydrid mit gesättigter wäßriger Natriumsulfatlösung (20 ml) zerstört. Der sich bildende Niederschlag wurde über Celite abfiltriert. Das Filtrat wurde eingeengt und mit Essigester extarhiert (3 $\times$ 100 ml). Die organische Phase wurde abgetrennt, getrocknet (Natriumsulfat) und im Vakuum eingeengt. Säulenchromatographische Reinigung (Kieselgel, 50% Essigester in n-Hexan) ergab 2,5 g Methyl-(8-thiophen-3-yl-1,4-dioxa-spiro[4.5]dec-8-yl)-amin (43%; weißer, niedrigschmelzender Feststoff).

**Beispiel 16:** 1,1-[3-Methylamino-3-(3-thienyl)pentamethylen]-1,3,4,9-tetrahydropyrano[3,4-b]indol Citrat

Beispiel 16 wurde analog zu Beispiel 14 aus 4-Methylamino-4-thiophen-3-yl-cyclohexanon und 2-(1H-Indol-3-yl)-ethanol hergestellt.

**Beispiel 17:** 1,1-[3-Methylamino-3-(3-thienyl)pentamethylen]-1,3,4,9-tetrahydropyrano[3,4-b]-6-fluorindol Citrat

Beispiel 17 wurde analog zu Beispiel 15 aus 4-Methylamino-4-thiophen-3-yl-cyclohexanon und 2-(5-Fluor-1 H-indol-3-yl)-ethanol hergestellt.

**Untersuchungen zur Wirksamkeit der erfindungsgemäßen Verbindungen:**

**Messung der ORL1-Bindung**

Die Cyclohexan-Derivate der allgemeinen Formel I wurden in einem Rezeptorbindungsassay mit $^3$H-Nociceptin/Orphanin FQ mit Membranen von rekombinanten CHO-ORL1 Zellen untersucht. Dieses Testsystem wurde gemäß der von Ardati et al. (Mol. Pharmacol., 51, 1997, S. 816-824) vorgestellten Methode durchgeführt. Die Konzentration von $^3$H-Nociceptin/Orphanin FQ betrug bei diesen Versuchen 0.5 nM. Die Bindungsassays wurden mit je 20 $\mu$g Membranprotein je 200 $\mu$l Ansatz in 50 mM Hepes, pH 7,4, 10 mM $MgCl_2$ und 1 mM EDTA durchgeführt. Die Bindung an den ORL1-Rezeptor wurde unter Verwendung von je 1 mg WGA-SPA Beads (Amersham-Pharmacia, Freiburg), durch einstündige Inkubation des Ansatzes bei RT und anschliessende Messung im Szintillationscounter Trilux (Wallac, Finnland), bestimmt. Die Affinität wird in Tabelle 1 als nanomolarer $K_i$-Wert in oder % Inhibition bei c=1 $\mu$M angegeben.

**Messung der $\mu$-Bindung**

Die Rezeptoraffinität zum humanen $\mu$-Opiatrezeptor wurde in einem homogenen Ansatz in Mikrotiterplatten bestimmt. Hierzu wurden Verdünnungsreihen des jeweils zu prüfenden substituierten Cyclohexyl-1,4-diamin-Derivates mit einer Rezeptormembranpräparation (15-40 $\mu$g Protein pro 250 $\mu$l Inkubationsansatz) von CHO-K1-Zellen, welche den humanen $\mu$-Opiatrezeptor exprimieren (RB-HOM-Rezeptormembran-Präparation der Firma NEN, Zaventem, Belgien) in Gegenwart von 1 nmol/l des radioaktiven Liganden [$^3$H]-Naloxon (NET719, Firma NEN, Zaventem, Belgien) sowie von 1 mg WGA-SPA-Beads (Wheat germ agglutinin SPA Beads der Firma Amersham/Pharmacia, Freiburg, Deutschland) in einem Gesamtvolumen von 250 µl für 90 Minuten bei Raumtemperatur inkubiert. Als Inkubationspuffer wurde 50 mmol/l Tris-HCl supplementiert mit 0,05 Gew.-% Natriumazid und mit 0,06 Gew.-% bovinem Serumalbumin verwendet. Zur Bestimmung der unspezifischen Bindung wurde zusätzlich 25 µmol/l Naloxon zugegeben. Nach Beendigung der neunzigminütigen Inkubationszeit wurden die Mikrotiterplatten für 20 Minuten bei 1000 g abzentrifugiert und die Radioaktivität in einem β-Counter (Microbeta-Trilux, Firma PerkinElmer Wallac, Freiburg, Deutschland) vermessen. Es wurde die prozentuale Verdrängung des radioaktiven Liganden aus seiner Bindung zum humanen µ-Opiatrezeptor bei einer Konzentration der Prüfsubstanzen von 1 µmol/l bestimmt und als prozentuale Hemmung (%Hemmung) der spezifischen Bindung angegeben. Teilweise wurden ausgehend von der prozentualen Verdrängung durch unterschiedliche Konzentrationen der zu prüfenden Verbindungen der allgemeinen Formel I $IC_{50}$ Hemmkonzentrationen berechnet, die eine 50-prozentige Verdrängung des radioaktiven Liganden bewirken. Durch Umrechnung mittels der Cheng-Prusoff-Beziehung wurden Ki-Werte für die Prüfsubstanzen erhalten.

| Beispiel-Nummer | ORL1 Ki (nM) oder % Hemmung | ORµ_Nal Ki (nM) oder % Hemmung |
|---|---|---|
| 1 | 1,60 | 2,80 |
| 3 | 49% | 140,00 |
| 4 | 0,49 | 0,08 |
| 5 | 29% | 210,00 |
| 6 | 37% | 47% |
| 7 | 0,56 | 0,27 |
| 8 | 0,26 | 0,12 |
| 10 | 0,66 | 0,09 |
| 11 | 41% | 53% |
| 12 | 59% | 150,00 |
| 13 | 0,61 | 0,08 |

**Analgesieprüfung im Tail-Flick-Test an der Maus**

Die Mäuse wurden jeweils einzeln in einen Testkäfig gesetzt und die Schwanzbasis dem fokussierten Wärmestrahl einer elektrischen Lampe (Tail-flick-Typ 50/08/1.bc, Labtec, Dr. Hess) ausgesetzt. Die Lampenintensität wurde so eingestellt, daß die Zeit vom Einschalten der Lampe bis zum plötzlichen Wegzucken des Schwanzes (Schmerzlatenz) bei unbehandelten Mäusen 3 bis 5 Sekunden betrug. Vor der Applikation der Lösungen enthaltend die erfindungsgemäße Verbindung bzw. der jeweiligen Vergleichslösungen wurden die Mäuse innerhalb von fünf Minuten zweimal vorgetestet und der Mittelwert dieser Messungen als Vortestmittelwert berechnet.

Die Lösungen der erfindungsgemäßen Verbindung der allgemeinen Formel I sowie die Vergleichslösungen wurden dann intravenös appliziert. Die Schmerzmessung wurde jeweils 10, 20, 40 und 60 Minuten nach der intravenösen Applikation durchgeführt. Die analgetische Wirkung wurde als Zunahme der Schmerzlatenz (% des maximal möglichen antinociceptiven Effektes) nach der folgenden Formel bestimmt:

$$[(T_1-T_0)/(T_2-T_0)] \times 100$$

Hierbei ist die Zeit $T_0$ die Latenzzeit vor der Applikation, die Zeit $T_1$ die Latenzzeit nach der Applikation der Wirkstoffkombination und die Zeit $T_2$ die maximale Expositionsdauer (12 Sekunden).

| **Beispiel Nr.** | **Tail Flick** (Maus, i.v.) $ED_{50}$ |
|---|---|
| **7** | 3,5 μg/kg |
| **10** | 0,028 mg/kg |
| **13** | 0,027 mg/kg |

Für Beispiel 7 wird als weitere Wirkung an der Maus beobachtet, dass es Muskelrelaxation und Narkose auslöst.

Beispiel 7 weist eine gegenüber reinen μ-Opioiden (wie Morphin) reduzierte Platzpräferenz auf (Platzpräferenz siehe Tzschentke, T.M. 1998, *Prog. Neurobiol.,* 56, 613 672).

**Entzugsspringen an der Maus**

Jumping-test an der Maus: Versuch zur Bestimmung der körperlichen Abhängigkeit nach der Methode von Saelens et al, 1971

Die Testsubstanzen werden insgesamt 7x über zwei Tage intraperitoneal appliziert. 5 Applikationen erfolgen am ersten Tag um 9:00, 10:00, 11:00, 13:00 und 15:00 Uhr und am zweiten Tag um 9:00 und 11:00 Uhr. Die ersten 3 Applikationen werden in aufsteigenden Dosierungen (Dosierungsschema) gegeben und dann weiter in der Dosierung der dritten. Der Entzug wird 2 Stunden nach der letzten

Substanzaplikation mit Naloxon 30 mg/kg (i.p.) präzipititert. Unmittelbar danach werden die Tiere einzeln in durchsichtige Beobachtungsboxen (Höhe 40 cm, Durchmesser 15 cm) gesetzt und die Sprungreaktionen über 15 Minuten in jeweils 5-Minuten- Perioden gezählt. Morphin wird in einer Dosierung als Vergleich/Standard mitgeführt.

Die Quantifizierung des Entzugs erfolgt über die Anzahl der Sprünge 0 bis 10 min. nach Naloxonapplikation. Die Anzahl der Tiere pro Gruppe mit mehr als 10 Sprüngen/10 min wird bestimmt und als "% positive Tiere" dokumentiert. Außerdem wird die durchschnittliche Sprung-Frequenz in der Gruppe errechnet. Pro Gruppe werden 12 Tiere eingesetzt

Die folgende Abbildung zeigt die dosisabhängige Ausprägung des Naloxoninduzierten Entzugsspringen an der Maus für Beispiel 7.

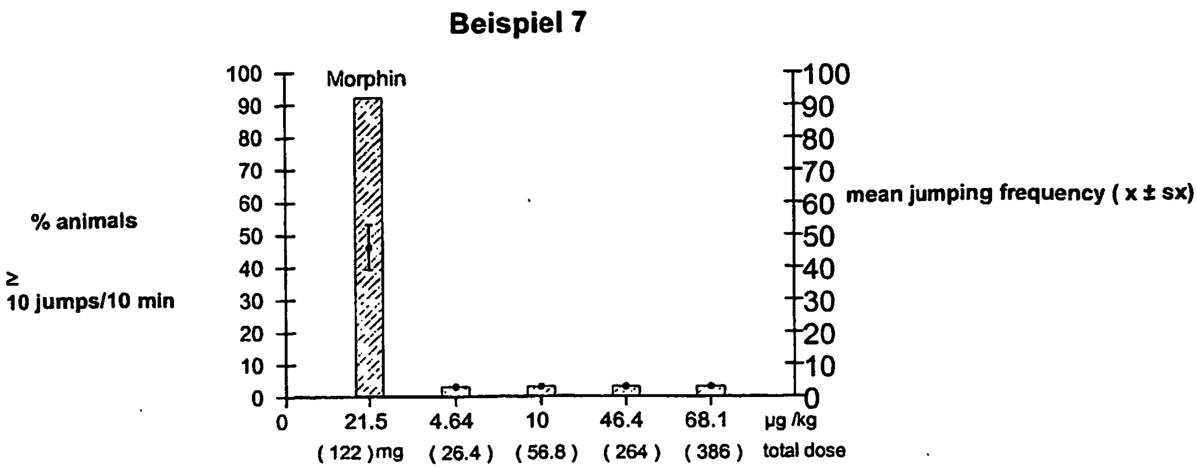


Das Entzugsspringen wird vollständig unterdrückt.

**Patentansprüche**

1. Spirocyclische Cyclohexan-Derivate der allgemeinen Formel I,

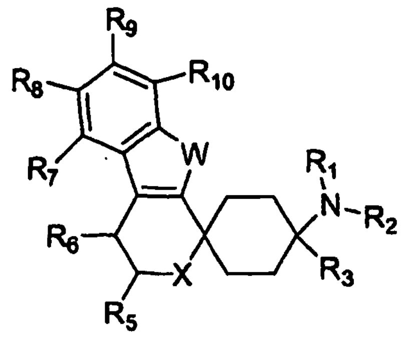

I

, worin

$R^1$ und $R^2$, unabhängig voneinander für H; CHO; $C_{1-5}$-Alkyl jeweils gesättigt oder ungesättigt, verzweigt oder unverzweigt, einfach oder mehrfach substituiert oder unsubstituiert; $C_{3-8}$-Cycloalkyl, jeweils gesättigt oder ungesättigt, einfach oder mehrfach substituiert oder unsubstituiert; oder über $C_{1-3}$-Alkyl gebundenes Aryl, $C_{3-8}$-Cycloalkyl oder Heteroaryl, jeweils einfach oder mehrfach substituiert oder unsubstituiert, stehen;

oder die Reste $R^1$ und $R^2$ zusammen für $CH_2CH_2OCH_2CH_2$, $CH_2CH_2NR^{11}$ $CH_2CH_2$ oder $(CH_2)_{3-6}$ stehen,

wobei $R^{11}$ H; $C_{1-5}$-Alkyl, jeweils gesättigt oder ungesättigt, verzweigt oder unverzweigt, einfach oder mehrfach substituiert oder unsubstituiert; $C_{3-8}$-Cycloalkyl, jeweils gesättigt oder ungesättigt, einfach oder mehrfach substituiert oder unsubstituiert; Aryl-, oder Heteroaryl, jeweils einfach oder mehrfach substituiert oder unsubstituiert; oder über $C_{1-3}$-Alkyl gebundenes Aryl, $C_{3-8}$-Cycloalkyl oder Heteroaryl, jeweils einfach oder mehrfach substituiert oder unsubstituiert, bedeutet;

$R^3$ für Heteroaryl, jeweils einfach oder mehrfach substituiert, steht;

W für $NR^4$, O oder S steht

und

$R^4$ für H; $C_{1-5}$-Alkyl, gesättigt oder ungesättigt, verzweigt oder unverzweigt, unsubstituiert oder einfach oder mehrfach substituiert; Aryl, oder Heteroaryl, jeweils substituiert oder unsubstituiert; über eine $C_{1-3}$-Alkyl-Gruppe gebundenes Aryl, Heteroaryl oder Cycloalkyl, jeweils einfach oder mehrfach substituiert oder unsubstituiert; $COR^{12}$ ; $SO_2R^{12}$ steht,

wobei $R^{12}$ H; $C_{1-5}$-Alkyl, jeweils gesättigt oder ungesättigt, verzweigt oder unverzweigt, einfach oder mehrfach substituiert oder unsubstituiert; $C_{3-8}$-Cycloalkyl, jeweils gesättigt oder ungesättigt, einfach oder mehrfach substituiert oder unsubstituiert; Aryl-, oder Heteroaryl, jeweils einfach oder mehrfach substituiert oder unsubstituiert; oder über $C_{1-3}$-Alkyl gebundenes Aryl, $C_{3-8}$-Cycloalkyl oder Heteroaryl, jeweils einfach oder mehrfach substituiert oder unsubstituiert; $OR^{13}$; $NR^{14}R^{15}$ bedeutet;

$R^5$ für =O; H; $COOR^{13}$, $CONR^{13}$, $OR^{13}$; $C_{1-5}$-Alkyl, gesättigt oder ungesättigt, verzweigt oder unverzweigt, unsubstituiert oder einfach oder mehrfach substituiert; $C_{3-8}$-Cycloalkyl, gesättigt oder ungesättigt, unsubstituiert oder einfach oder mehrfach substituiert; Aryl-, oder Heteroaryl, unsubstituiert oder einfach oder mehrfach substituiert; oder über $C_{1-3}$-Alkyl gebundenes Aryl, $C_{3-8}$-Cycloalkyl oder Heteroaryl, unsubstituiert oder einfach oder mehrfach substituiert, steht;

$R^6$ für H; F, Cl, $NO_2$, $CF_3$, $OR^{13}$, $SR^{13}$, $SO_2R^{13}$, $SO_2OR^{13}$, CN, $COOR^{13}$, $NR^{14}R^{15}$; $C_{1-5}$-Alkyl, gesättigt oder ungesättigt, verzweigt oder unverzweigt, unsubstituiert oder einfach oder mehrfach substituiert; $C_{3-8}$-Cycloalkyl, gesättigt oder ungesättigt, unsubstituiert oder einfach oder mehrfach substituiert; Aryl-, oder Heteroaryl, unsubstituiert oder einfach oder mehrfach substituiert; oder über $C_{1-3}$-Alkyl gebundenes Aryl, $C_{3-8}$-Cycloalkyl oder Heteroaryl, unsubstituiert oder einfach oder mehrfach substituiert, steht;

oder $R^5$ und $R^6$ gemeinsam $(CH_2)_n$ mit n = 2, 3, 4, 5 oder 6 bedeuten, wobei einzelne Wasserstoffatome auch durch F, Cl, Br, 1, $NO_2$, $CF_3$, $OR^{13}$, CN oder $C_{1-5}$-Alkyl ersetzt sein können;

$R^7$, $R^8$, $R^9$ und $R^{10}$ unabhängig voneinander für

H, F, Cl, Br, I, $NO_2$, $CF_3$, $OR^{13}$, $SR^{13}$, $SO_2R^{13}$, $SO_2OR^{13}$, CN, $COOR^{13}$, $NR^{14}R^{15}$; $C_{1-5}$-Alkyl, $C_{3-8}$-Cycloalkyl, unsubstituiert oder einfach oder mehrfach substituiert; Aryl-, oder Heteroaryl, unsubstituiert oder einfach oder mehrfach substituiert; oder über $C_{1-3}$-Alkyl gebundenes Aryl, $C_{3-8}$-Cycloalkyl oder Heteroaryl, unsubstituiert oder einfach oder mehrfach substituiert, stehen;

wobei $R^{13}$ H; $C_{1-5}$-Alkyl jeweils gesättigt oder ungesättigt, verzweigt oder unverzweigt, unsubstituiert oder einfach oder mehrfach substituiert; $C_{3-8}$-Cycloalkyl, jeweils gesättigt oder ungesättigt, unsubstituiert oder einfach oder mehrfach substituiert; Aryl-, oder Heteroaryl, unsubstituiert oder einfach oder mehrfach substituiert; oder über $C_{1-3}$-Alkyl

gebundenes Aryl, $C_{3-8}$-Cycloalkyl oder Heteroaryl, unsubstituiert oder einfach oder mehrfach substituiert, bedeutet; $R^{14}$ und $R^{15}$ unabhängig voneinander H; $C_{1-5}$-Alkyl , jeweils gesättigt oder ungesättigt, verzweigt oder unverzweigt, unsubstituiert oder einfach oder mehrfach substituiert; oder $C_{3-8}$-Cycloalkyl, jeweils gesättigt oder ungesättigt, unsubstituiert oder einfach oder mehrfach substituiert; Aryl-, oder Heteroaryl, unsubstituiert oder einfach oder mehrfach substituiert; oder über $C_{1-3}$-Alkyl gebundenes Aryl, $C_{3-8}$-Cycloalkyl oder Heteroaryl, unsubstituiert oder einfach oder mehrfach substituiert, bedeuten;
oder $R^{14}$ und $R^{15}$ zusammen $CH_2CH_2OCH_2CH_2$, $CH_2CH_2NR^{16}CH_2CH_2$ oder $(CH_2)_{3-6}$ bilden,
wobei $R^{16}$ H; $C_{1-5}$-Alkyl gesättigt oder ungesättigt, verzweigt oder unverzweigt, unsubstituiert oder einfach oder mehrfach substituiert, bedeutet;
X für O, S, SO, $SO_2$ oder $NR^{17}$ steht;
$R^{17}$ für H; $C_{1-5}$-Alkyl, gesättigt oder ungesättigt, verzweigt oder unverzweigt; $COR^{12}$ oder $SO_2R^{12}$ steht,
in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren oder Kationen.

2. Spirocyclische Cyclohexan-Derivate gemäß Anspruch 1, **dadurch gekennzeichnet, dass** $R^1$ und $R^2$ unabhängig voneinander für H, $C_{1-5}$-Alkyl, verzweigt oder unverzweigt, gesättigt oder ungesättigt, unsubstituiert oder einfach oder mehrfach substituiert, oder CHO stehen, und/oder
$R^3$ für Heteroaryl, jeweils unsubstituiert oder ein- oder mehrfach substituiert, steht

3. Spirocyclische Cyclohexan-Derivate gemäß einem der Ansprüche 1 oder 2, **dadurch gekennzeichnet, dass** $R^5$ für H, $C_{1-5}$-Alkyl, verzweigt oder unverzweigt, unsubstituiert oder einfach oder mehrfach substituiert oder $COOR^{13}$ steht und $R^6$ H oder $C_{1-5}$-Alkyl bedeutet.

4. Spirocyclische Cyclohexan-Derivate gemäß einem der Ansprüche 1-3, **dadurch gekennzeichnet, dass** $R^7$, $R^8$, $R^9$ und $R^{10}$ unabhängig voneinander für H; $C_{1-5}$-Alkyl, verzweigt oder unverzweigt, unsubstituiert oder ein- oder mehrfach substituiert; F, Cl, Br, I, $CF_3$, OH, $OCH_3$, $NH_2$, COOH, $COOCH_3$, $NHCH_3$ oder $N(CH_3)_2$ oder $NO_2$ stehen.

5. Spirocyclische Cyclohexan-Derivate gemäß Anspruch 1, **dadurch gekennzeichnet, dass**
W für $NR^4$, O oder S steht und X O, S, SO, $SO_2$ oder $NR^{17}$ bedeutet,
$R^1$ und $R^2$ unabhängig voneinander für H; $C_{1-4}$Alkyl, verzweigt oder unverzweigt, ein- oder mehrfach substituiert oder unsubstituiert; oder CHO stehen
$R^3$ für Heteroaryl, unsubstituiert oder einfach oder mehrfach substituiert;
$R^4$ für H; $C_{1-3}$-Alkyl, ein- oder mehrfach substituiert oder unsubstituiert;
$CO(CH_2)_mH$ mit m = 0 bis 2, steht, und/oder
$R^5$ und $R^6$ jeweils für H stehen und/oder
$R^7$, $R^8$, $R^9$ und $R^{10}$ unabhängig voneinander für H; $C_{1-5}$-Alkyl, $OC_{1-3}$-Alkyl, jeweils verzweigt oder unverzweigt, gesättigt oder ungesättigt, unsubstituiert oder ein- oder mehrfach substituiert; F, Cl, Br, I, $CF_3$, OH, SH, $SCH_3$, $OCH_3$, $NH_2$, COOH, $COOCH_3$, $NHCH_3$ oder $N(CH_3)_2$ oder $NO_2$, stehen.

6. Spirocyclische Cyclohexan-Derivate gemäß einem der Ansprüche 1-5, **dadurch gekennzeichnet, dass** $R^1$ und $R^2$ unabhängig voneinander H oder $CH_3$ bedeuten, wobei $R^1$ und $R^2$ nicht gleichzeitig H bedeuten.

7. Spirocyclische Cyclohexan-Derivate gemäß einem der Ansprüche 1-5, **dadurch gekennzeichnet, dass**
W für $NR^4$ steht und X O bedeutet,
$R^1$ und $R^2$ unabhängig voneinander für H; $C_{1-4}$-Alkyl, verzweigt oder unverzweigt, ein- oder mehrfach substituiert oder unsubstituiert; oder CHO stehen
$R^3$ für Heteroaryl, unsubstituiert oder einfach oder mehrfach substituiert, steht $R^4$ für H; $C_{1-3}$-Alkyl, ein- oder mehrfach substituiert oder unsubstituiert; $CO(CH_2)_mH$ mit m = 0 bis 2, steht,
$R^5$ und $R^6$ jeweils für H stehen und/oder
$R^7$, $R^8$, $R^9$ und $R^{10}$ unabhängig voneinander für H; $C_{1-5}$-Alkyl, $OC_{1-3}$-Alkyl, jeweils verzweigt oder unverzweigt, gesättigt oder ungesättigt, unsubstituiert oder ein- oder mehrfach substituiert; F, Cl, Br, I, $CF_3$, OH, SH, $SCH_3$, $OCH_3$, $NH_2$, COOH, $COOCH_3$, $NHCH_3$ oder $N(CH_3)_2$ oder $NO_2$, stehen.

8. Spirocyclische Cyclohexan-Derivate gemäß einem der Ansprüche 1-6, **dadurch gekennzeichnet, dass** $R^3$ Thienyl oder Pyridyl bedeutet

9. Spirocyclische Cyclohexan-Derivate gemäß einem der Ansprüche 1-8, **dadurch gekennzeichnet, dass**

der Rest $R^5$ für H, $CH_3$, $COOCH_3$ oder $CH_2OH$ steht
der Rest $R^6$ für H steht
die Reste $R^7$, $R^8$, $R^9$ und $R^{10}$ für H stehen
oder
einer der Reste $R^7$, $R^8$, $R^9$ und $R^{10}$ für H; $C_{1-5}$-Alkyl, verzweigt oder unverzweigt, unsubstituiert oder ein- oder mehrfach substituiert; F, Cl, Br, I, OH, $OCH_3$, COOH, $COOCH_3$, $NH_2$, $NHCH_3$ oder $N(CH_3)_2$ oder $NO_2$ steht, während die übrigen Reste H bedeuten,
oder
zwei der Reste $R^7$ $R^8$, $R^9$ und $R^{10}$ unabhängig voneinander für H; $C_{1-5}$-Alkyl, verzweigt oder unverzweigt, unsubstituiert oder ein- oder mehrfach substituiert; F, Cl, Br, I, OH, $OCH_3$, COOH, $COOCH_3$, $NH_2$, $NHCH_3$ oder $N(CH_3)_2$ oder $NO_2$ stehen, während die übrigen Reste H bedeuten.

**10.** Spirocyclische Cyclohexan-Derivate gemäß einem der Ansprüche 1-9, **dadurch gekennzeichnet, dass** $R^1$ und $R^2$ $CH_3$ bedeuten und $R^3$ Thienyl oder Pyridyl bedeutet.

**11.** Spirocyclische Cyclohexan-Derivate gemäß einem der Ansprüche 1-10, **dadurch gekennzeichnet, dass** W für $NR^4$, X für O und $R^4$ für H, $CH_3$, $C_2H_5$, Acetyl, Phenyl, Benzyl oder $COR^{12}$ steht.

**12.** Spirocyclische Cyclohexan-Derivate gemäß einem der Ansprüche 1-11 aus der Gruppe

1,1-[3-Dimethylamino-3-(pyridin-2-yl)pentamethylen]-3,4-dihydro-1H-2,9-diazafluoren
2-Acetyl-1,1-[3-dimethylamino-3-(pyridin-2-yl)pentamethylen]-3,4-dihydro-1H-2,9-diazafluoren
1,1-[3-Dimethylamino-3-(pyridin-2-yl)pentamethylen]-3,4-dihydro-1H-2-oxa-9-thiafluoren
1,1-[3-Dimethylamino-3-(pyridin-2-yl)pentamethylen]-1,3,4,9-tetrahydropyrano-[3,4-b]indol Hemicitrat, unpolareres Diastereoisomer
1,1-[3-Dimethylamino-3-(pyridin-2-yl)pentamethylen]-1,3,4,9-tetrahydropyrano-[3,4-b]indol Citrat, polareres Diastereoisomer
1,1-[3-Dimethylamino-3-(2-thienyl)pentamethylen]-1,3,4,9-tetra-hydropyrano[3,4-b]indol Dimethansulfonat; polareres Diastereomer
1,1-[3-Dimethylamino-3-(2-thienyl)pentamethylen]-1,3,4,9-tetra-hydropyrano[3,4-b]indol Citrat; unpolareres Diastereomer
1,1-[3-Dimethylamino-3-(3-thienyl)pentamethylen]-1,3,4,9-tetra-hydropyrano[3,4-b]indol Hemicitrat; unpolareres Diastereomer
1,1-[3-Dimethylamino-3-(3-thienyl )pentamethylen]-1,3,4,9-tetra-hydropyrano[3,4-b]indol Citrat; polareres Diastereomer
1,1-[3-Dimethylamino-3-(2-thienyl)pentamethylen]-1,3,4,9-tetra-hydropyrano[3,4-b]-6-fluorindol Hemicitrat; unpolareres Diastereomer
1,1-[3-Dimethylamino-3-(2-thienyl)pentamethylen]-1,3,4,9-tetra-hydropyrano[3,4-b]-6-fluorindol Citrat; polareres Diastereomer
1,1-[3-Dimethylamino-3-(3-thienyl)pentamethylen]-1,3;4,9-tetra-hydropyrano[3,4-b]-6-fluorindol Dimethansulfonat; polareres Diastereomer
1,1-[3-Dimethylamino-3-(3-thienyl)pentamethylen]-1,3,4,9-tetra-hydropyrano[3,4-b]-6-fluorindol Hemicitrat; unpolareres Diastereomer
1,1-[3-Methylamino-3-(2-thienyl)pentamethylen]-1,3,4,9-tetrahydro-pyrano[3,4-b]indol Citrat
1,1-[3-Methylamino-3-(2-thienyl)pentamethylen]-1,3,4,9-tetrahydro-pyrano[3,4-b]-6-fluorindol Citrat
1,1-[3-Methylamino-3-(3-thienyl)pentamethylen]-1,3,4,9-tetrahydro-pyrano[3,4-b]indol Citrat
1,1-[3-Methylamino-3-(3-thienyl)pentamethylen]-1,3,4,9-tetrahydro-pyrano[3,4-b]-6-fluorindol Citrat

gegebenenfalls auch als Gemisch.

**13.** Verfahren zur Herstellung von spirocyclischen Cyclohexanderivaten gemäß einem der Ansprüche 1-12, **dadurch gekennzeichnet, dass** ein Edukt der allgemeinen Formel A

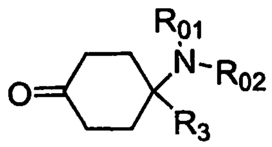

A

wobei die Reste $R^{01}$ und $R^{02}$ die für $R^2$ angegebene Bedeutung haben und zusätzlich für eine Schutzgruppe stehen können, unter Zugabe von Säure, oder deren Trimethylsilylester, beispielsweise Trifluormethansulfonsäuretrimethylsilylester, Trifluormethansulfonsäure, Essigsäure, Phosphorsäure, Methansulfonsäure oder Trifluoressigsäure, in einem geeigneten Lösungsmittel, beispielsweise Dichlorethan, Dichlormethan, Chloroform, Acetonitril, Diethylether oder Nitromethan, mit einem Edukt der allgemeinen Formel B

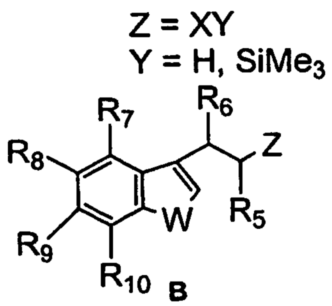

B

umgesetzt wird, wobei die Reste $R^1$-$R^{10}$ die in Anspruch 1 angegebenen Bedeutungen haben.

**14.** Verfahren zur Herstellung von spirocyclischen Cyclohexanderivaten gemäß Anspruch 1, bei denen X $NR^{17}$ und $R^{17}$ $COR^{12}$ oder $SO_2R^{12}$ bedeutet, **dadurch gekennzeichnet, dass** ein spirocyclisches Cyclohexanderivat, bei dem X NH bedeutet, unter Zugabe von Base, beispielsweise Triethylamin, mit einem Anhydrid oder einem Säurechlorid umgesetzt wird, vorzugsweise unter Mikrowelleneinstrahlung.

**15.** Verfahren zur Herstellung von spirocyclischen Cyclohexanderivaten gemäß Anspruch 1, bei denen X SO oder $SO_2$ bedeutet, **dadurch gekennzeichnet, dass** ein spirocyclisches Cyclohexanderivat, bei dem X S bedeutet, mit Hilfe eines Oxidationsmittels, beispielsweise $H_2O_2$, oxidiert wird.

**16.** Verfahren zur Herstellung von spirocyclischen Cyclohexanderivaten der allgemeinen Formel Ib

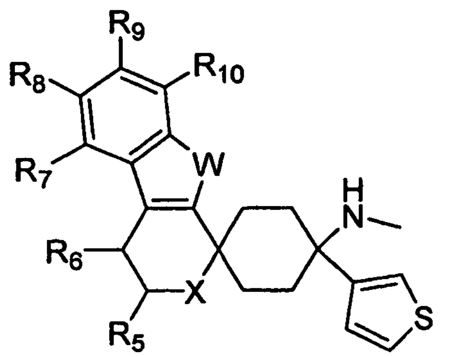

,

Ib

**dadurch gekennzeichnet, dass** Thiophen-3-yl-acetonitril mit einem Acrylester oder einem 3-Brompropionsäureester umgesetzt, verseift, decarboxyliert und mit Schutzgruppen versehen wird, die Nitrilgruppe durch Verseifung in die Carbonsäure und dann in das Isocyanat überführt wird, anschließend mit einem Reduktionsmittel, beispielsweise Lithiumaluminiumhydrid, umgesetzt und nach Entfernung der Schutzgruppen unter Zugabe von Säure oder deren Trimethylsilylester, beispielsweise Trifluormethansulfonsäuretrimethylsilylester, Trifluormethansulfonsäure, Essigsäure, Phosphorsäure, Methansulfonsäure oder Trifluoressigsäure, in einem geeigneten Lösungsmittel, beispielsweise Dichlorethan, Dichlormethan, Chloroform, Acetonitril, Diethylether oder Nitromethan, mit einem Edukt der allgemeinen Formel B

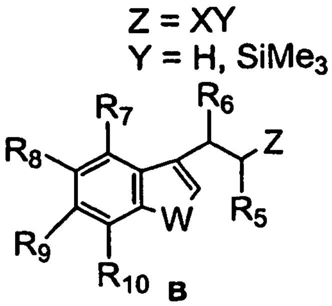

B

umgesetzt wird.

**17.** Arzneimittel enthaltend wenigstens ein spirocyclisches Cyclohexan -Derivat gemäß einem der Ansprüche 1 bis 12, gegebenenfalls in Form seines Razemats, der reinen Stereoisomeren, insbesondere Enantiomeren und Diastereomeren, in einem beliebigen Mischungsverhältnis; in Form seiner Säuren oder seiner Basen oder in Form seiner Salze, insbesondere der physiologisch verträglichen Salze oder Salze physiologisch verträglicher Säuren oder Kationen; oder in Form seiner Solvate, insbesondere der Hydrate, sowie gegebenenfalls enthaltend geeignete Zusatz- und/oder Hilfsstoffe und/oder gegebenenfalls weiterer Wirkstoffe.

**18.** Verwendung eines spirocyclischen Cyclohexan-Derivates gemäß einem der Ansprüche 1 bis 12, gegebenenfalls in Form seines Razemats, der reinen Stereoisomeren, insbesondere Enantiomeren und Diastereomeren, in einem beliebigen Mischungsverhältnis; in Form seiner Säuren oder seiner Basen oder in Form seiner Salze, insbesondere der physiologisch verträglichen Salze oder Salze physiologisch verträglicher Säuren oder Kationen; oder in Form seiner Solvate, insbesondere der Hydrate; zur Herstellung eines Arzneimittels zur Behandlung von Schmerz, insbesondere von akutem, neuropathischem oder chronischem Schmerz.

**19.** Verwendung eines spirocyclisches Cyclohexan-Derivats gemäß einem der Ansprüche 1 bis 12 zur Herstellung eines Arzneimittels zur Behandlung von Alkohol- und/oder Drogen- und/oder Medikamentenmißbrauch und/oder -abhängigkeit, als Muskelrelaxanz oder Anesthetikum, bzw. zur Coadministration bei Behandlung mit einem opioiden Analgetikum oder mit einem Anesthetikum, zur Behandlung von Entzugserscheinungen und/oder zur. Reduzierung des Suchtpotentials von Opioiden.

**20.** Verwendung eines spirocyclisches Cyclohexan-Derivats gemäß einem der Ansprüche 1 bis 12 zur Herstellung eines Arzneimittels zur Behandlung von Angstzuständen, von Stress und mit Stress verbundenen Syndromen, Depressionen, Epilepsie, Alzheimer Erkrankung, seniler Demenz, allgemeinen kognitiven Dysfunktionen, Lern- und Gedächtnis-Störungen (als Nootropikum), sexuellen Dysfunktionen, cardiovaskulären Erkrankungen, Hypotension, Hypertension, Tinitus, Pruritus, Migräne, Schwerhörigkeit, mangelnder Darmmotilität, gestörter Nahrungsaufnahme, Anorexie, Fettsucht, lokomotorischen Störungen, Diarrhoe, Kachexie, Harninkontinenz bzw. als Antikonvulsivum, zur Diurese oder Antinatriurese, Anxiolyse, zur Modulation der Bewegungsaktivität, zur Modulation der Neurotransmitter-Ausschüttung und Behandlung damit verbundener neurodegenerativer Erkrankungen.

## Claims

**1.** Spirocyclic cyclohexane derivatives of the general formula I

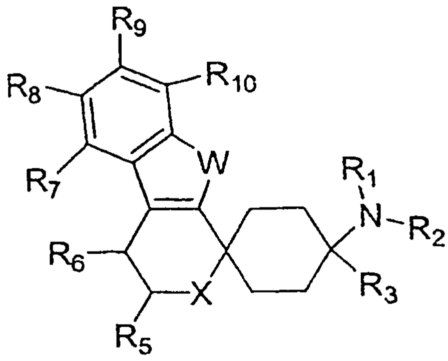

I

wherein

$R^1$ and $R^2$ independently of one another represent H; CHO; $C_{1-5}$-alkyl, in each case saturated or unsaturated, branched or unbranched, mono- or polysubstituted or unsubstituted; $C_{3-8}$-cycloalkyl, in each case saturated or unsaturated, mono- or polysubstituted or unsubstituted; or aryl, $C_{3-8}$-cycloalkyl or heteroaryl, bonded via $C_{1-3}$-alkyl and in each case mono- or polysubstituted or unsubstituted;

or the radicals $R^1$ and $R^2$ together represent $CH_2CH_2OCH_2CH_2$, $CH_2CH_2NR^{11}CH_2CH_2$ or $(CH_2)_{3-6}$,

wherein $R^{11}$ denotes H; $C_{1-5}$-alkyl, in each case saturated or unsaturated, branched or unbranched, mono- or polysubstituted or unsubstituted; $C_{3-8}$-cycloalkyl, in each case saturated or unsaturated, mono- or polysubstituted or unsubstituted; aryl or heteroaryl, in each case mono- or polysubstituted or unsubstituted; or aryl, $C_{3-8}$-cycloalkyl or heteroaryl, bonded via $C_{1-3}$-alkyl and in each case mono- or polysubstituted or unsubstituted;

$R^3$ represents heteroaryl, in each case mono- or polysubstituted;

W represents $NR^4$, O or S

and

$R^4$ represents H; $C_{1-5}$-alkyl, saturated or unsaturated, branched or unbranched, unsubstituted or mono- or polysubstituted; aryl or heteroaryl, in each case substituted or unsubstituted; or aryl, heteroaryl or cycloalkyl, bonded via a $C_{1-3}$-alkyl group and in each case mono- or polysubstituted or unsubstituted; $COR^{12}$; $SO_2R^{12}$,

wherein $R^{12}$ denotes H; $C_{1-5}$-alkyl, in each case saturated or unsaturated, branched or unbranched, mono- or polysubstituted or unsubstituted; $C_{3-8}$-cycloalkyl, in each case saturated or unsaturated, mono- or polysubstituted or unsubstituted; aryl or heteroaryl, in each case mono- or polysubstituted or unsubstituted; or aryl, $C_{3-8}$-cycloalkyl or heteroaryl, bonded via $C_{1-3}$-alkyl and in each case mono- or polysubstituted or unsubstituted; $OR^{13}$; $NR^{14}R^{15}$;

$R^5$ represents =O; H; $COOR^{13}$, $CONR^{13}$, $OR^{13}$; $C_{1-5}$-alkyl, saturated or unsaturated, branched or unbranched, unsubstituted or mono- or polysubstituted; $C_{3-8}$-cycloalkyl, saturated or unsaturated, unsubstituted or mono- or polysubstituted; aryl or heteroaryl, unsubstituted or mono- or polysubstituted; or aryl, $C_{3-8}$-cycloalkyl or heteroaryl, bonded via $C_{1-3}$-alkyl and unsubstituted or mono- or polysubstituted;

$R^6$ represents H; F, Cl, $NO_2$, $CF_3$, $OR^{13}$, $SR^{13}$, $SO_2R^{13}$, $SO_2OR^{13}$, CN, $COOR^{13}$ , $NR^{14}R^{15}$; $C_{1-5}$-alkyl, saturated or unsaturated, branched or unbranched, unsubstituted or mono- or polysubstituted; $C_{3-8}$-cycloalkyl, saturated or unsaturated, unsubstituted or mono- or polysubstituted; aryl or heteroaryl, unsubstituted or mono- or polysubstituted; or aryl, $C_{3-8}$-cycloalkyl or heteroaryl, bonded via $C_{1-3}$-alkyl and unsubstituted or mono- or polysubstituted;

or $R^5$ and $R^6$ together denote $(CH_2)_n$ where n = 2, 3, 4, 5 or 6, wherein individual hydrogen atoms can also be replaced by F, Cl, Br, I, $NO_2$, $CF_3$, $OR^{13}$, CN or $C_{1-5}$-alkyl;

$R^7$, $R^8$, $R^9$ and $R^{10}$ independently of one another represent

H, F, Cl, Br, I, $NO_2$, $CF_3$, $OR^{13}$, $SR^{13}$, $SO_2R^{13}$, $SO_2OR^{13}$, CN, $COOR^{13}$, $NR^{14}R^{15}$; $C_{1-5}$-alkyl, $C_{3-8}$-cycloalkyl, unsubstituted or mono- or polysubstituted; aryl or heteroaryl, unsubstituted or mono- or polysubstituted; or aryl, $C_{3-8}$-cycloalkyl or heteroaryl, bonded via $C_{1-3}$-alkyl and unsubstituted or mono- or polysubstituted;

wherein $R^{13}$ denotes H; $C_{1-5}$-alkyl, in each case saturated or unsaturated, branched or unbranched, unsubstituted or mono- or polysubstituted; $C_{3-8}$-cycloalkyl, in each case saturated or unsaturated, unsubstituted or mono- or polysubstituted; aryl or heteroaryl, unsubstituted or mono- or polysubstituted; or aryl, $C_{3-8}$-cycloalkyl or heteroaryl, bonded via $C_{1-3}$-alkyl and in each case unsubstituted or mono- or polysubstituted;

$R^{14}$ and $R^{15}$ independently of one another denote H; $C_{1-5}$-alkyl, in each case saturated or unsaturated, branched or unbranched, unsubstituted or mono- or polysubstituted; or $C_{3-8}$-cycloalkyl, in each case saturated or unsaturated, unsubstituted or mono- or polysubstituted; aryl or heteroaryl, unsubstituted or mono- or polysubstituted; or aryl, $C_{3-8}$-cycloalkyl or heteroaryl, bonded via $C_{1-3}$-alkyl and in each case unsubstituted or mono- or polysubstituted;

or $R^{14}$ and $R^{15}$ together form $CH_2CH_2OCH_2CH_2$, $CH_2CH_2NR^{16}CH_2CH_2$ or $(CH_2)_{3-6}$,

wherein $R^{16}$ denotes H; $C_{1-5}$-alkyl, saturated or unsaturated, branched or unbranched, unsubstituted or mono- or polysubstituted;
X represents O, S, SO, $SO_2$ or $NR^{17}$;
$R^{17}$ represents H; $C_{1-5}$-alkyl, saturated or unsaturated, branched or unbranched; $COR^{12}$ or $SO_2R^{12}$,
in the form of the racemate; of the enantiomers, diastereomers, mixtures of the enantiomers or diastereomers or of an individual enantiomer or diastereomer; of the bases and/or salts of physiologically acceptable acids or cations.

2. Spirocyclic cyclohexane derivatives according to claim 1, **characterized in that**
$R^1$ and $R^2$ independently of one another represent H, $C_{1-5}$-alkyl, branched or unbranched, saturated or unsaturated, unsubstituted or mono- or polysubstituted, or CHO, and/or
$R^3$ represents heteroaryl, in each case unsubstituted or mono- or polysubstituted.

3. Spirocyclic cyclohexane derivatives according to one of claims 1 or 2, **characterized in that** $R^5$ represents H, $C_{1-5}$-alkyl, branched or unbranched, unsubstituted or mono- or polysubstituted, or $COOR^{13}$ and $R^6$ denotes H or $C_{1-5}$-alkyl.

4. Spirocyclic cyclohexane derivatives according to one of claims 1-3, **characterized in that** $R^7$, $R^8$, $R^9$ and $R^{10}$ independently of one another represent H; $C_{1-5}$-alkyl, branched or unbranched, unsubstituted or mono- or polysubstituted; F, Cl, Br, I, $CF_3$, OH, $OCH_3$, $NH_2$, COOH, $COOCH_3$, $NHCH_3$ or N $(CH_3)_2$ or $NO_2$.

5. Spirocyclic cyclohexane derivatives according to claim 1, **characterized in that**
W represents $NR^4$, O or S and X denotes O, S, SO, $SO_2$ or $NR^{17}$,
$R^1$ and $R^2$ independently of one another represent H; $C_{1-4}$-alkyl, branched or unbranched, mono- or polysubstituted or unsubstituted; or CHO;
$R^3$ represents heteroaryl, unsubstituted or mono- or polysubstituted;
$R^4$ represents H; $C_{1-3}$-alkyl, mono- or polysubstituted or unsubstituted; $CO(CH_2)_mH$ where m = 0 to 2, and/or
$R^5$ and $R^6$ each represent H and/or
$R^7$, $R^8$, $R^9$ and $R^{10}$ independently of one another represent H; $C_{1-5}$-alkyl, $OC_{1-3}$-alkyl, in each case branched or unbranched, saturated or unsaturated, unsubstituted or mono- or polysubstituted; F, Cl, Br, I, $CF_3$ , OH, SH, $SCH_3$ , $OCH_3$ , $NH_2$, COOH, $COOCH_3$, $NHCH_3$ or $N(CH_3)_2$ or $NO_2$.

6. Spirocyclic cyclohexane derivatives according to one of claims 1-5, **characterized in that** $R^1$ and $R^2$ independently of one another denote H or $CH_3$, wherein $R^1$ and $R^2$ do not simultaneously denote H.

7. Spirocyclic cyclohexane derivatives according to one of claims 1-5, **characterized in that**
W represents $NR^4$ and X denotes O,
$R^1$ and $R^2$ independently of one another represent H; $C_{1-4}$-alkyl, branched or unbranched, mono- or polysubstituted or unsubstituted; or CHO
$R^3$ represents heteroaryl, unsubstituted or mono- or polysubstituted,
$R^4$ represents H; $C_{1-3}$-alkyl, mono- or polysubstituted or unsubstituted; $CO(CH_2)_mH$ where m = 0 to 2,
$R^5$ and $R^6$ each represent H and/or
$R^7$, $R^8$, $R^9$ and $R^{10}$ independently of one another represent H; $C_{1-5}$-alkyl, $OC_{1-3}$-alkyl, in each case branched or unbranched, saturated or unsaturated, unsubstituted or mono- or polysubstituted; F, Cl, Br, I, $CF_3$, OH, SH, $SCH_3$, $OCH_3$, $NH_2$, COOH, $COOCH_3$, $NHCH_3$ or $N(CH_3)_2$ or $NO_2$

8. Spirocyclic cyclohexane derivatives according to one of claims 1-6, **characterized in that** $R^3$ denotes thienyl or pyridyl.

9. Spirocyclic cyclohexane derivatives according to one of claims 1-8, **characterized in that**
the radical $R^5$ represents H, $CH_3$, $COOCH_3$ or $CH_2OH$
the radical $R^6$ represents H
the radicals $R^7$, $R^8$, $R^9$ and $R^{10}$ represent H
or
one of the radicals $R^7$, $R^8$, $R^9$ and $R^{10}$ represents H; $C_{1-5}$-alkyl, branched or unbranched, unsubstituted or mono- or polysubstituted; F, Cl, Br, I, OH, $OCH_3$, COOH, $COOCH_3$, $NH_2$, $NHCH_3$ or $N(CH_3)_2$ or $NO_2$, while the remaining radicals denote H,
or
two of the radicals $R^7$, $R^8$, $R^9$ and $R^{10}$ independently of one another represent H; $C_{1-5}$-alkyl, branched or unbranched,

unsubstituted or mono- or polysubstituted; F, Cl, Br, I, OH, $OCH_3$, COOH, $COOCH_3$, $NH_2$, $NHCH_3$ or $N(CH_3)_2$ or $NO_2$, while the remaining radicals denote H.

**10.** Spirocyclic cyclohexane derivatives according to one of claims 1-9, **characterized in that** $R^1$ and $R^2$ denote $CH_3$ and $R^3$ denotes thienyl or pyridyl.

**11.** Spirocyclic cyclohexane derivatives according to one of claims 1-10, **characterized in that** W represents $NR^4$, X represents O and $R^4$ represents H, $CH_3$, $C_2H_5$, acetyl, phenyl, benzyl or $COR^{12}$.

**12.** Spirocyclic cyclohexane derivatives according to one of claims 1-11, from the group consisting of

1,1-[3-dimethylamino-3-(pyridin-2-yl)pentamethylene]-3,4-dihydro-1H-2,9-diazafluorene
2-acetyl-1,1-[3-dimethylamino-3-(pyridin-2-yl)pentamethylene]-3,4-dihydro-1H-2,9-diazafluorene
1,1-[3-dimethylamino-3-(pyridin-2-yl)pentamethylene]-3,4-dihydro-1H-2-oxa-9-thiafluorene
1,1-[3-dimethylamino-3-(pyridin-2-yl)pentamethylene]-1,3,4,9-tetrahydropyrano[3,4-b]indole hemicitrate, less polar diastereoisomer
1,1-[3-dimethylamino-3-(pyridin-2-yl)pentamethylene]-1,3,4,9-tetrahydropyrano[3,4-b]indole citrate, more polar diastereoisomer
1,1-[3-dimethylamino-3-(2-thienyl)pentamethylene]-1,3,4,9-tetrahydropyrano[3,4-b]indole dimethanesulfonate; more polar diastereomer
1,1-[3-dimethylamino-3-(2-thienyl)pentamethylene]-1,3,4,9-tetrahydropyrano[3,4-b]indole citrate; less polar diastereomer
1,1-[3-dimethylamino-3-(3-thienyl)pentamethylene]-1,3,4,9-tetrahydropyrano[3,4-b]indole hemicitrate; less polar diastereomer
1,1-[3-dimethylamino-3-(3-thienyl)pentamethylene]-1,3,4,9-tetrahydropyrano[3,4-b]indole citrate; more polar diastereomer
1,1-[3-dimethylamino-3-(2-thienyl)pentamethylene]-1,3,4,9-tetrahydropyrano[3,4-b]-6-fluoroindole hemicitrate; less polar diastereomer
1,1-[3-dimethylamino-3-(2-thienyl)pentamethylene]-1,3,4,9-tetrahydropyrano[3,4-b]-6-fluoroindole citrate; more polar diastereomer
1,1-[3-dimethylamino-3-(3-thienyl)pentamethylene]-1,3,4,9-tetrahydropyrano[3,4-b]-6-fluoroindole dimethanesulfonate; more polar diastereomer
1,1-[3-dimethylamino-3-(3-thienyl)pentamethylene]-1,3,4,9-tetrahydropyrano[3,4-b]-6-fluoroindole hemicitrate; less polar diastereomer
1,1-[3-methylamino-3-(2-thienyl)pentamethylene]-1,3,4,9-tetrahydropyrano[3,4-b]indole citrate
1,1-[3-methylamino-3-(2-thienyl)pentamethylene]-1,3,4,9-tetrahydropyrano[3,4-b]-6-fluoroindole citrate
1,1-[3-methylamino-3-(3-thienyl)pentamethylene]-1,3,4,9-tetrahydropyrano[3,4-b]indole citrate
1,1-[3-methylamino-3-(3-thienyl)pentamethylene]-1,3,4,9-tetrahydropyrano[3,4-b]-6-fluoroindole citrate

optionally also as a mixture.

**13.** Process for the preparation of spirocyclic cyclohexane derivatives according to one of claims 1-12, **characterized in that** an educt of the general formula A

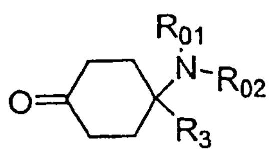

**A**

wherein the radicals $R^{01}$ and $R^{02}$ have the meaning given for $R^2$ and can additionally represent a protective group, is reacted, with the addition of acid or trimethylsilyl ester thereof, for example trifluoromethanesulfonic acid trimethylsilyl ester, trifluoromethanesulfonic acid, acetic acid, phosphoric acid, methanesulfonic acid or trifluoroacetic, in a suitable solvent, for example dichloroethane, methylene chloride, chloroform, acetonitrile, diethyl ether or nitromethane, with an educt of the general formula B

Z = XY
Y = H, $SiMe_3$

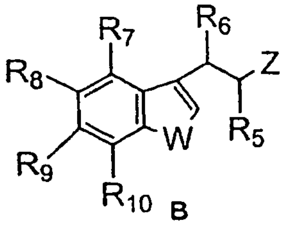

B

wherein the radicals $R^1$-$R^{10}$ have the meanings given in claim 1.

**14.** Process for the preparation of spirocyclic cyclohexane derivatives according to claim 1, in which X denotes $NR^{17}$ and $R^{17}$ denotes $COR^{12}$ or $SO_2R^{12}$, **characterized in that** a spirocyclic cyclohexane derivative in which X denotes NH is reacted, with the addition of base, for example triethylamine, with an anhydride or an acid chloride, preferably under microwave irradiation.

**15.** Process for the preparation of spirocyclic cyclohexane derivatives according to claim 1, in which X denotes SO or $SO_2$, **characterized in that** a spirocyclic cyclohexane derivative in which X denotes S is oxidized with the aid of an oxidizing agent, for example $H_2O_2$.

**16.** Process for the preparation of spirocyclic cyclohexane derivatives of the general formula Ib

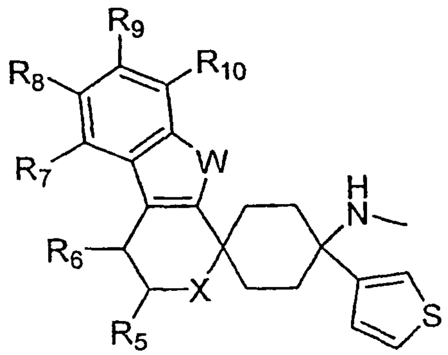

Ib

**characterized in that** thiophen-3-yl-acetonitrile is reacted with an acrylic ester or a 3-bromopropionic acid ester, the product is hydrolyzed, decarboxylated and provided with protective groups, the nitrile group is converted by hydrolysis into the carboxylic acid and then into the isocyanate, and the product is subsequently reacted with a reducing agent, for example lithium aluminium hydride, and, after removal of the protective groups, is reacted, with addition of acid or trimethylsilyl ester thereof, for example trifluoromethanesulfonic acid trimethylsilyl ester, trifluoromethanesulfonic acid, acetic acid, phosphoric acid, methanesulfonic acid or trifluoroacetic acid, in a suitable solvent, for example dichloroethane, methylene chloride, chloroform, acetonitrile, diethyl ether or nitromethane, with an educt of the general formula B

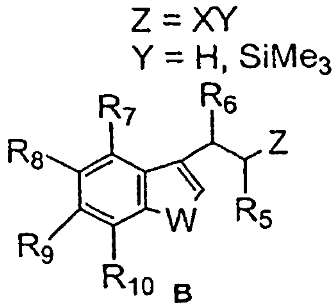

B

17. Medicament containing at least one spirocyclic cyclohexane derivative according to one of claims 1 to 12, optionally in the form of its racemate, the pure stereoisomers, in particular enantiomers and diastereomers, in any desired mixing ratio; in the form of its acids or its bases or in the form of its salts, in particular the physiologically acceptable salts or salts of physiologically acceptable acids or cations; or in the form of its solvates, in particular the hydrates, and optionally containing suitable additives and/or auxiliary substances and/or optionally further active compounds.

18. Use of a spirocyclic cyclohexane derivative according to one of claims 1 to 12, optionally in the form of its racemate, the pure stereoisomers, in particular enantiomers and diastereomers, in any desired mixing ratio; in the form of its acids or its bases or in the form of its salts, in particular the physiologically acceptable salts or salts of physiologically acceptable acids or cations; or in the form of its solvates, in particular the hydrates; for the preparation of a medicament for treatment of pain, in particular acute, neuropathic or chronic pain.

19. Use of a spirocyclic cyclohexane derivative according to one of claims 1 to 12 for the preparation of a medicament for treatment of alcohol and/or drug and/or medicament abuse and/or dependency, as a muscle relaxant or anaesthetic, or for co-administration with treatment with an opioid analgesic or with an anaesthetic, for treatment of withdrawal symptoms and/or for reducing the addiction potential of opioids.

20. Use of a spirocyclic cyclohexane derivative according to one of claims 1 to 12 for the preparation of a medicament for treatment of anxiety states, of stress and syndromes associated with stress, depressions, epilepsy, Alzheimer's disease, senile dementia, general cognitive dysfunctions, learning and memory disorders (as a nootropic), sexual dysfunctions, cardiovascular diseases, hypotension, hypertension, tinnitus, pruritus, migraine, impaired hearing, lack of intestinal motility, impaired food intake, anorexia, obesity, locomotor disorders, diarrhoea, cachexia, urinary incontinence, or as an anticonvulsive, for diuresis or antinatriuresis, anxiolysis, for modulation of movement activity, for modulation of neurotransmitter secretion and treatment of neurodegenerative diseases associated therewith.

## Revendications

1. Dérivés spirocycliques de cyclohexane de formule générale I,

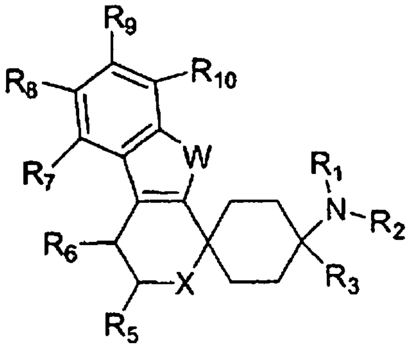

I

dans laquelle

$R^1$ et $R^2$ indépendamment l'un de l'autre, représentent H ; CHO ; un reste alkyle en $C_1$ à $C_5$, dans chaque cas saturé ou non saturé, ramifié ou non ramifié, substitué une ou plusieurs fois ou non substitué ; un reste cycloalkyle en $C_3$ à $C_8$, dans chaque cas saturé ou non saturé, substitué une ou plusieurs fois ou non substitué ; ou un reste, lié par l'intermédiaire d'un groupe alkyle en $C_1$ à $C_3$, aryle, cycloalkyle en $C_3$ à $C_8$ ou hétéroaryle, chacun substitué une ou plusieurs fois ou non substitué ;

ou bien les restes $R^1$ et $R^2$ forment ensemble un groupe $CH_2CH_2OCH_2CH_2$, $CH_2CH_2NR^{11}CH_2CH_2$ ou $(CH_2)_{3-6}$,

$R^{11}$ représentant H ; un reste alkyle en $C_1$ à $C_5$, dans chaque cas saturé ou non saturé, ramifié ou non ramifié, substitué une ou plusieurs fois ou non substitué ; un reste cycloalkyle en $C_3$ à $C_8$, dans chaque cas saturé ou non saturé, substitué une ou plusieurs fois ou non substitué ; un reste aryle ou hétéroaryle, chacun substitué une ou plusieurs fois ou non substitué ; ou bien un reste, lié par l'intermédiaire d'un groupe alkyle en $C_1$ à $C_3$, aryle, cycloalkyle en $C_3$ à $C_8$ ou hétéroaryle, chacun substitué une ou plusieurs fois ou non substitué ;

$R^3$ représente un reste hétéroaryle, dans chaque cas substitué une ou plusieurs fois ;

W représente $NR^4$, O ou S

et

$R^4$ représente H ; un reste alkyle en $C_1$ à $C_5$, saturé ou non saturé, ramifié ou non ramifié, non substitué ou substitué une ou plusieurs fois; un reste aryle ou hétéroaryle, chacun substitué ou non substitué ; un reste, lié par l'intermédiaire d'un groupe alkyle en $C_1$ à $C_3$, aryle, hétéroaryle ou cycloalkyle, chacun substitué une ou plusieurs fois ou non substitué ; un groupe $COR^{12}$; un groupe $SO_2R^{12}$,

$R^{12}$ représentant H ; un reste alkyle en $C_1$ à $C_5$, dans chaque cas saturé ou non saturé, ramifié ou non ramifié, substitué une ou plusieurs fois ou non substitué ; un reste cycloalkyle en $C_3$ à $C_8$, dans chaque cas saturé ou non saturé, substitué une ou plusieurs fois ou non substitué ; un reste aryle ou hétéroaryle, chacun substitué une ou plusieurs fois ou non substitué ; ou un reste, lié par l'intermédiaire d'un groupe alkyle en $C_1$ à $C_3$, aryle, cycloalkyle en $C_3$ à $C_8$ ou hétéroaryle, chacun substitué une ou plusieurs fois ou non substitué ; un groupe $OR^{13}$ ; $NR^{14}R^{15}$ ;

$R^5$ représente =O ; H ; un groupe $COOR^{13}$, $CONR^{13}$, $OR^{13}$ ; un reste alkyle en $C_1$ à $C_5$, saturé ou non saturé, ramifié ou non ramifié, non substitué ou substitué une ou plusieurs fois ; un reste cycloalkyle en $C_3$ à $C_8$, ramifié ou non ramifié, non substitué ou substitué une ou plusieurs fois ; un reste aryle ou hétéroaryle, non substitué ou substitué une ou plusieurs fois ; ou un reste, lié par l'intermédiaire d'un groupe alkyle en $C_1$ à $C_3$, aryle, cycloalkyle en $C_3$ à $C_8$ ou hétéroaryle, non substitué ou substitué une ou plusieurs fois ;

$R^6$ représente H ; F, Cl, $NO_2$, $CF_3$, $OR^{13}$, $SR^{13}$, $SO_2R^{13}$, $SO_2OR^{13}$, CN, $COOR^{13}$, $NR^{14}R^{15}$ ; un reste alkyle en $C_1$ à $C_5$, saturé ou non saturé, ramifié ou non ramifié, non substitué ou substitué une ou plusieurs fois ; un reste cycloalkyle en $C_3$ à $C_8$, saturé ou non saturé, non substitué ou substitué une ou plusieurs fois ; un reste aryle ou hétéroaryle, non substitué ou substitué une ou plusieurs fois ; ou un reste, lié par l'intermédiaire d'un groupe alkyle en $C_1$ à $C_3$, aryle, cycloalkyle en $C_3$ à $C_8$ ou hétéroaryle, non substitué ou substitué une ou plusieurs fois ;

ou bien $R^5$ et $R^6$ forment ensemble un groupe $(CH_2)_n$ dans lequel n est égal à 2, 3, 4, 5 ou 6, des atomes individuels d'hydrogène pouvant aussi être remplacés par F, Cl, Br, I, $N0_2$, $CF_3$, $OR^{13}$ , CN ou alkyle en $C_1$ à $C_5$;

$R^7$, $R^8$, $R^9$ et $R^{10}$ représentent, indépendamment les uns des autres, H, F, Cl, Br, I, $NO_2$, $CF_3$, $OR^{13}$, $SR^{13}$, $SO_2R^{13}$, $SO_2OR^{13}$, CN, $COOR^{13}$, $NR^{14}R^{15}$ ; un reste alkyle en $C_1$ à $C_5$, cycloalkyle en $C_3$ à $C_8$, non substitué ou substitué une ou plusieurs fois ; un reste aryle ou hétéroaryle, non substitué ou substitué une ou plusieurs fois ; ou un reste, lié par l'intermédiaire d'un groupe alkyle en $C_1$ à $C_3$, aryle, cycloalkyle en $C_3$ à $C_8$ ou hétéroaryle, non substitué ou substitué une ou plusieurs fois ;

$R^{13}$ représentant H ; un reste alkyle en $C_1$ à $C_5$, dans chaque cas saturé ou non saturé, ramifié ou non ramifié, non substitué ou substitué une ou plusieurs fois ; un reste cycloalkyle en $C_3$ à $C_8$, dans chaque cas saturé ou non saturé, non substitué ou substitué une ou plusieurs fois ; un reste aryle ou hétéroaryle, non substitué ou substitué une ou plusieurs fois ; ou un reste, lié par l'intermédiaire d'un groupe alkyle en $C_1$ à $C_3$, aryle, cycloalkyle en $C_3$ à $C_8$ ou hétéroaryle, non substitué ou substitué une ou plusieurs fois ;

$R^{14}$ et $R^{15}$ représentent, indépendamment l'un de l'autre, H, un reste alkyle en $C_1$ à $C_5$, chacun saturé ou non saturé, ramifié ou non ramifié, non substitué ou substitué une ou plusieurs fois ; ou un reste cycloalkyle en $C_3$ à $C_8$, dans chaque cas saturé ou non saturé, non substitué ou substitué une ou plusieurs fois ; un reste aryle ou hétéroaryle, non substitué ou substitué une ou plusieurs fois ; ou un reste, lié par l'intermédiaire d'un groupe alkyle en $C_1$ à $C_3$, aryle, cycloalkyle en $C_3$ à $C_8$ ou hétéroaryle, non substitué ou substitué une ou plusieurs fois ;

ou bien $R^{14}$ et $R^{15}$ forment ensemble un groupe $CH_2CH_2OCH_2CH_2$, $CH_2CH_2NR^{16}CH_2CH_2$ ou $(CH_2)_{3-6}$,

$R^{16}$ représentant H ; un reste alkyle en $C_1$ à $C_5$, saturé ou non saturé, ramifié ou non ramifié, non substitué ou substitué une ou plusieurs fois ;

X représente O, S, SO, $SO_2$ ou $NR^{17}$ ;

$R^{17}$ représentant H ; un reste alkyle en $C_1$ à $C_5$, saturé ou non saturé, ramifié ou non ramifié ; un groupe $COR^{12}$ ou $SO_2R^{12}$,

sous forme du racémate ; des énantiomères, des diastéréoisomères, de mélanges des énantiomères ou des diastéréoisomères, ou d'un énantiomère ou diastéréoisomère individuel ; des bases et/ou des sels d'acides ou de cations physiologiquement acceptables.

2. Dérivés spirocycliques de cyclohexane suivant la revendication 1, **caractérisés en ce que** $R^1$ et $R^2$ représentent, indépendamment l'un de l'autre, H, un reste alkyle en $C_1$ à $C_5$, ramifié ou non ramifié, saturé ou non saturé, non substitué ou substitué une ou plusieurs fois, ou un groupe CHO, et/ou $R^3$ représente un reste hétéroaryle, dans chaque cas non substitué ou substitué une ou plusieurs fois.

3. Dérivés spirocycliques de cyclohexane suivant l'une des revendications 1 ou 2, **caractérisés en ce que** $R^5$ représente H, un reste alkyle en $C_1$ à $C_5$, ramifié ou non ramifié, non substitué ou substitué une ou plusieurs fois ou un groupe $COOR^{13}$, et $R^6$ représente H ou un reste alkyle en $C_1$ à $C_5$.

4. Dérivés spirocycliques de cyclohexane suivant l'une des revendications 1 à 3, **caractérisés en ce que** $R^7$, $R^8$, $R^9$ et $R^{10}$ représentent, indépendamment les uns des autres, H ; un reste alkyle en $C_1$ à $C_5$, ramifié ou non ramifié, non substitué ou substitué une ou plusieurs fois ; F, Cl, Br, I, $CF_3$, OH, $OCH_3$, $NH_2$, COOH, $COOCH_3$, $NHCH_3$ ou $N(CH_3)_2$ ou $NO_2$.

5. Dérivés spirocycliques de cyclohexane suivant la revendication 1, **caractérisés en ce que**
W représente un groupe $NR^4$, O ou S, et X représente O, S, SO, $SO_2$ ou $NR^{17}$,
$R^1$ et $R^2$ représentent, indépendamment l'un de l'autre, H ; un reste alkyle en $C_1$ à $C_4$, ramifié ou non ramifié, substitué une ou plusieurs fois ou non substitué ; ou un groupe CHO,
$R^3$ représente un reste hétéroaryle, non substitué ou substitué une ou plusieurs fois,
$R^4$ représente H ; un reste alkyle en $C_1$ à $C_3$, substitué une ou plusieurs fois ou non substitué ; un groupe CO $(CH_2)_mH$, dans lequel m a une valeur de 0 à 2, et/ou
$R^5$ et $R^6$ représentent chacun H, et/ou
$R^7$, $R^8$, $R^9$ et $R^{10}$ représentent, indépendamment les uns des autres, H ; un reste alkyle en $C_1$ à $C_5$, 0(alkyle en $C_1$ à $C_3$), chacun ramifié ou non ramifié, saturé ou non saturé, non substitué ou substitué une ou plusieurs fois ; F, Cl, Br, I, $CF_3$, OH, SH, $SCH_3$, $OCH_3$, $NH_2$, COOH, $COOCH_3$, $NHCH_3$ ou $N(CH_3)_2$ ou $NO_2$.

6. Dérivés spirocycliques de cyclohexane suivant l'une des revendications 1 à 5, **caractérisés en ce que** $R^1$ et $R^2$ représentent, indépendamment l'un de l'autre, H ou $CH_3$, $R^1$ et $R^2$ ne représentant pas simultanément H.

7. Dérivés spirocycliques de cyclohexane suivant l'une des revendications 1 à 5, **caractérisés en ce que**
W représente un groupe $NR^4$ et X représente O,
$R^1$ et $R^2$ représentent, indépendamment l'un de l'autre, H ; un reste alkyle en $C_1$ à $C_4$, ramifié ou non ramifié, substitué une ou plusieurs fois ou non substitué ; ou un groupe CHO,
$R^3$ représente un reste hétéroaryle, non substitué ou substitué une ou plusieurs fois,
$R^4$ représente H ; un reste alkyle en $C_1$ à $C_3$, substitué une ou plusieurs fois ou non substitué ; un groupe CO $(CH_2)_mH$, dans lequel m a une valeur de 0 à 2,
$R^5$ et $R^6$ représentent chacun H, et/ou
$R^7$, $R^8$, $R^9$ et $R^{10}$ représentent, indépendamment les uns des autres, H ; un reste alkyle en $C_1$ à $C_5$, O (alkyle en $C_1$ à $C_3$), chacun ramifié ou non ramifié, saturé ou non saturé, non substitué ou substitué une ou plusieurs fois ; F, Cl, Br, I, $CF_3$, OH, SH, $SCH_3$, $OCH_3$, $NH_2$, COOH, $COOCH_3$, $NHCH_3$ ou N $(CH_3)_2$ ou $NO_2$.

8. Dérivés spirocycliques de cyclohexane suivant l'une des revendications 1 à 6, **caractérisés en ce que** $R^3$ représente un reste thiényle ou pyridyle.

9. Dérivés spirocycliques de cyclohexane suivant l'une des revendications 1 à 8, **caractérisés en ce que**
le reste $R^5$ représente H, $CH_3$, $COOCH_3$ ou $CH_2OH$,
le reste $R^6$ représente H,
les restes $R^7$, $R^8$, $R^9$ et $R^{10}$ représentent H
ou bien
l'un des restes $R^7$, $R^8$, $R^9$ et $R^{10}$ représente H ; un reste alkyle en $C_1$ à $C_5$, ramifié ou non ramifié, non substitué ou substitué une ou plusieurs fois ; F, Cl, Br, I, OH , OCH), COOH, $COOCH_3$, $NH_2$, $NHCH_3$ ou $N(CH_3)_2$ ou $NO_2$, tandis que les autres restes représentent H,
ou bien
deux des restes $R^7$, $R^8$, $R^9$ et $R^{10}$ représentent, indépendamment l'un de l'autre, H ; un reste alkyle en $C_1$ à $C_5$, ramifié ou non ramifié, non substitué ou substitué une ou plusieurs fois ; F, Cl, Br, I, OH, $OCH_3$, COOH, $COOCH_3$,

$NH_2$, $NHCH_3$ ou N $(CH_3)_2$ ou $NO_2$, tandis que les autres restes représentent H.

**10.** Dérivés spirocycliques de cyclohexane suivant l'une des revendications 1 à 9, **caractérisés en ce que** $R^1$ et $R^2$ représentent $CH_3$ et $R^3$ est un reste thiényle ou pyridyle.

**11.** Dérivés spirocycliques de cyclohexane suivant l'une des revendications 1 à 10, **caractérisés en ce que** W représente $NR^4$, X représente O et $R^4$ représente H, $CH_3$, $C_2H_5$, un reste acétyle, phényle, benzyle ou un groupe $COR^{12}$.

**12.** Dérivés spirocycliques de cyclohexane suivant l'une des revendications 1 à 11, du groupe des composés

1,1-[3-diméthylamino-3-(pyridine-2-yl)pentaméthylène]-3,4-dihydro-1H-2,9-diazafluorène,
2-acétyl-1,1-[3-diméthylamino-3-(pyridine-2-yl)pentaméthylène]-3,4-dihydro-1H-2,9-diazafluorène,
1,1-[3-diméthylamino-3-(pyridine-2-yl)pentaméthylène]-3,4-dihydro-1H-2-oxa-9-thiafluorène,
1,1-[3-diméthylamino-3-(pyridine-2-yl)pentaméthylène]-1,3,4,9-tétrahydropyranno[3,4-b]indole, hémicitrate, diastéréoisomère le moins polaire,
1,1-[3-diméthylamino-3-(pyridine-2-yl)pentaméthylène]-1,3,4,9-tétrahydropyranno[3,4-b]indole, citrate, diastéréoisomère le plus polaire,
1,1-[3-diméthylamino-3-(2-thiényl)pentaméthylène]-1,3,4,9-tétrahydropyranno[3,4-b]indole, diméthanesulfonate, diastéréoisomère le plus polaire,
1,1-[3-diméthylamino-3-(2-thiényl)pentaméthylène]-1,3,4,9-tétrahydropyranno[3,4-b]indole, citrate, diastéréoisomère le moins polaire,
1,1-[3-diméthylamino-3-(3-thiényl)pentaméthylène]-1,3,4,9-tétrahydropyranno[3,4-b]indole, hémicitrate, diastéréoisomère le moins polaire,
1,1-[3-diméthylamino-3-(3-thiényl)pentaméthylène]-1,3,4,9-tétrahydropyranno[3,4-b]indole, citrate, diastéréoisomère le plus polaire,
1,1-[3-diméthylamino-3-(2-thiényl)pentaméthylène]-1,3,4,9-tétrahydropyranno[3,4-b]-6-fluorindole, hémicitrate, diastéréoisomère le moins polaire,
1,1-[3-diméthylamino-3-(2-thiényl)pentaméthylène]-1,3,4,9-tétrahydropyranno[3,4-b]-6-fluorindole; citrate, diastéréoisomère le plus polaire,
1,1-[3-diméthylamino-3-(3-thiényl)pentaméthylène]-1,3,4,9-tétrahydropyranno[3,4-b]-6-fluorindole, diméthanesulfonate, diastéréoisomère le plus polaire,
1,1-[3-diméthylamino-3-(3-thiényl)pentaméthylène]-1,3,4,9-tétrahydropyranno[3,4-b]-6-fluorindole, hémicitrate, diastéréoisomère le moins polaire,
1,1-[3-méthylamino-3-(2-thiényl)pentaméthylène]-1,3,4,9-tétrahydropyranno[3,4-b]indole, citrate,
1,1-[3-méthylamino-3-(2-thiényl)pentaméthylène]-1,3,4,9-tétrahydropyranno[3,4-b]-6-fluorindole, citrate,
1,1-[3-méthylamino-3-(3-thiényl)pentaméthylène]-1,3,4,9-tétrahydropyranno[3,4-b]indole, citrate,
1,1-[3-méthylamino-3-(3-thiényl)pentaméthylène]-1,3,4,9-tétrahydropyranno[3,4-b]-6-fluorindole, citrate,

le cas échéant également sous forme de mélange.

**13.** Procédé de production de dérivés spirocycliques de cyclohexane suivant l'une des revendications 1 à 12, **caractérisé en ce qu'**un composé de départ de formule générale A

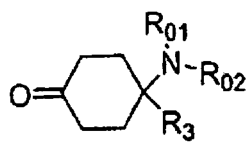

**A**

dans laquelle les restes $R^{01}$ et $R^{02}$ qui ont la définition indiquée pour $R^2$ peuvent en outre représenter un groupe protecteur, avec addition d'acide ou de son ester triméthylsilylique, par exemple l'ester triméthylsilylique de l'acide trifluorométhanesulfonique, l'acide trifluorométhanesulfonique, l'acide acétique, l'acide phosphorique, l'acide méthanesulfonique ou l'acide trifluoracétique, dans un solvant convenable, par exemple le dichloréthane, le dichlorométhane, le chloroforme, l'acétonitrile, l'éther de diéthyle ou le nitrométhane, est amené à réagir avec un composé de départ de formule générale B

Z = XY
Y = H, $SiMe_3$

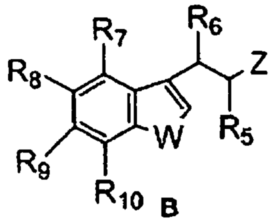

B

les restes $R_1$ à $R_{10}$ ayant les définitions indiquées dans la revendication 1.

**14.** Procédé de production de dérivés spirocycliques de cyclohexane suivant la revendication 1, dans lesquels X représente $NR^{17}$ et $R^{17}$ est un groupe $COR^{12}$ ou $SO_2R^{12}$, **caractérisé en ce qu'**un dérivé spirocyclique de cyclohexane, dans lequel X représente NH, est amené à réagir avec un anhydride ou un chlorure d'acide, avec addition d'une base, par exemple de triéthylamine, avantageusement sous irradiation par micro-ondes.

**15.** Procédé de production de dérivés spirocycliques de cyclohexane suivant la revendication 1, dans lesquels X représente SO ou $SO_2$, **caractérisé en ce qu'**un dérivé spirocyclique de cyclohexane dans lequel X représente S est oxydé à l'aide d'un agent oxydant, par exemple $H_2O_2$.

**16.** Procédé de production de dérivés spirocycliques de cyclohexane de formule générale Ib

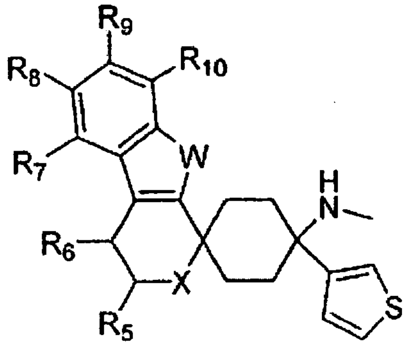

Ib

**caractérisé en ce que** du thiophène-3-yl-acétonitrile est amené à réagir avec un ester acrylique ou un ester d'acide 3-bromopropionique, saponifié, décarboxylé et pourvu de groupes protecteurs, le groupe nitrile est converti par saponification en acide carboxylique puis en l'isocyanate, après quoi il est amené à réagir avec un agent réducteur, par exemple l'hydrure de lithium et d'aluminium, puis amené à réagir, après élimination des groupes protecteurs avec addition d'acide ou de son ester triméthylsilylique, par exemple d'ester triméthylsilylique d'acide trifluorométhanesulfonique, d'acide trifluorométhanesulfonique, d'acide acétique, d'acide phosphorique, d'acide méthanesulfonique ou d'acide trifluoracétique, dans un solvant convenable, par exemple le dichloréthane, le dichlorométhane, le chloroforme, l'acétonitrile, l'éther de diéthyle ou le nitrométhane, avec un composé de départ de formule générale B

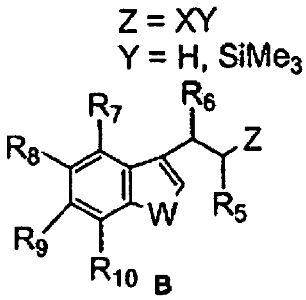

B

**17.** Médicament contenant au moins un dérivé spirocyclique de cyclohexane suivant l'une des revendications 1 à 12, éventuellement sous forme de son racémate, des stéréoisomères purs, en particulier des énantiomères et des diastéréoisomères, dans un rapport de mélange quelconque ; sous forme de ses acides ou de ses bases ou sous forme de ses sels, en particulier des sels physiologiquement acceptables ou de sels d'acides ou de cations physiologiquement acceptables ; ou sous forme de ses produits de solvatation, en particulier des hydrates, contenant eux-mêmes le cas échéant des additifs et/ou des substances auxiliaires convenables et/ou éventuellement d'autres substances actives.

**18.** Utilisation d'un dérivé spirocyclique de cyclohexane suivant l'une des revendications 1 à 12, éventuellement sous forme de son racémate, des stéréoisomères purs, en particulier des énantiomères et des diastéréoisomères, dans un rapport de mélange quelconque ; sous forme de ses acides ou de ses bases ou sous forme de ses sels, en particulier des sels physiologiquement acceptables ou de sels d'acides ou de cations physiologiquement acceptables ; ou sous forme de ses produits de solvatation, en particulier des hydrates ; pour la préparation d'un médicament destiné au traitement de la douleur, en particulier d'une douleur aiguë, neuropathique ou chronique.

**19.** Utilisation d'un dérivé spirocyclique de cyclohexane suivant l'une des revendications 1 à 12, pour la préparation d'un médicament destiné au traitement de l'abus d'alcool et/ou de drogues et/ou de médicaments et/ou de la dépendance à l'égard de l'alcool et/ou des drogues et/ou des médicaments, comme myorelaxant ou anesthésique, ou en vue d'une coadministration dans un traitement avec un analgésique opiacé ou avec un anesthésique, pour le traitement de phénomènes inflammatoires et/ou pour la réduction du potentiel de dépendance des opioïdes.

**20.** Utilisation d'un dérivé spirocyclique de cyclohexane suivant l'une des revendications 1 à 12, pour la préparation d'un médicament destiné au traitement d'états d'anxiété, de stress et de syndromes liés au stress, de dépressions, de l'épilepsie, de la maladie d'Alzheimer, de la démence sénile, de dysfonctionnements cognitifs généraux, de troubles de l'apprentissage et de la mémoire (comme nootrope), de dysfonctionnements sexuels, de maladies cardio-vasculaires, de l'hypotension, de l'hypertension, des acouphènes, du prurit, de la migraine, de la surdité, de l'insuffisance de motilité intestinale, de troubles de l'absorption de nourriture, de l'anorexie, de l'obésité, de troubles locomoteurs, de la diarrhée, de la cachexie, de l'incontinence d'urine ou comme anticonvulsifs, pour la diurèse ou l'antinatriurèse, pour l'anxiolyse, pour la modulation de l'activité motrice, pour la modulation de l'excrétion de neurotransmetteurs, et pour le traitement de maladies neurodégénératives qui y sont liées.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 466548 A
- WO 0290317 A
- US 4065573 A


### In der Beschreibung aufgeführte Nicht-Patentliteratur


- **MEUNIER et al.** *Nature,* 1995, vol. 377, 532-535
- **REINSCHEID et al.** *Science,* 1995, vol. 270, 792-794
- **MOGIL et al.** *Neuroscience,* 1996, vol. 75, 333-337
- **JENCK et al.** *Proc. Natl. Acad. Sci. USA,* 1997, vol. 94, 14854-14858
- **KING et al.** *Neurosci. Lett.,* 1997, vol. 223, 113-116
- **ABDULLA ; SMITH.** *J. Neurosci.,* 1998, vol. 18, 9685-9694
- **MANABE et al.** *Nature,* 1997, vol. 394, 577-581
- **NISHI et al.** *EMBO J.,* 1997, vol. 16, 1858-1864
- **CALO et al.** *Br.J. Pharmacol.,* 2000, vol. 129, 1261-1283
- **LEDNICER et al.** *J. Med. Chem.,* 1980, vol. 23, 424-430
- **ARDATI et al.** *Mol. Pharmacol.,* 1997, vol. 51, 816-824